# EUROPEAN PATENT SPECIFICATION

(11) **EP 3 791 895 B1**
(45) Date of publication and mention of the grant of the patent: **30.10.2024**
(21) Application number: 19800254.5
(22) Date of filing: 10.05.2019
(51) Int. Cl.: A61K 39/395, A61P 3/06, A61P 5/00

(54) **PREPARATIONS COMPRISING ANTI-PCSK9 ANTIBODIES AND USE THEREOF**
ZUBEREITUNGEN MIT ANTI-PCSK9-ANTIKÖRPERN UND IHRE VERWENDUNG
PRÉPARATIONS COMPRENANT DES ANTICORPS ANTI-PCSK9 ET LEUR UTILISATION

(30) Priority: 11.05.2018 CN 201810450088
(43) Date of publication of application: 17.03.2021
(73) Proprietor: Innovent Biologics (Suzhou) Co., Ltd., Suzhou, Jiangsu 215123 (CN)
(72) Inventor: WANG, Yinjue, Suzhou, Jiangsu 215123 (CN); LIU, Xiaolin, Suzhou, Jiangsu 215123 (CN); XIE, Ruixia, Suzhou, Jiangsu 215123 (CN)
(74) Representative: Sagittarius IP
(86) International application number: PCT/CN2019/086388
(87) International publication number: WO 2019/214707

(56) References cited:
- EP-A1- 3 560 961
- WO-A1-2012/154999
- WO-A1-2018/113781
- CN-A- 103 717 237
- CN-A- 103 841 992
- US-A1- 2013 189 277
- VIOLA MARGARIDA ET AL: "Subcutaneous delivery of monoclonal antibodies: How do we get there?", JOURNAL OF CONTROLLED RELEASE, ELSEVIER, AMSTERDAM, NL, vol. 286, 2 August 2018 (2018-08-02), pages 301 - 314, XP085478006, ISSN: 0168-3659, DOI: 10.1016/J.JCONREL.2018.08.001

## Description

### TECHNICAL FIELD

The present invention relates to the field of antibody preparations, and in particular to a pharmaceutical preparation comprising an antibody specifically binding to proprotein convertase subtilisin/kexin type 9 (PCSK9) (referred to as anti-PCSK9 antibody hereinafter) and/or an antibody fragment thereof. Furthermore, the present invention relates to therapeutic and diagnostic use of the preparation.

### BACKGROUND

Elevated cholesterol level in serum is an important risk factor resulting in cardiovascular events. At present, cholesterol-lowering drugs with multiple mechanisms of action have been launched or are under development. Among them, anti-PCSK9 monoclonal antibodies have drawn extensive attention because of their good safety and efficacy (Zhao Shuiping, et al., Chinese Guideline for the Management of Dyslipidemia in Adults (2016 Revised Edition). Chinese Circulation Journal 31(10): 937-953, 2016).

With high specificity in action, mild adverse reaction and other advantages, monoclonal antibody drugs have become the mainstream of drug development in many disease fields, and anti-PCSK9 monoclonal antibody drugs that reduce LDL-C by reducing LDLR endocytosis are a current research hotspot. With a remarkable effect in reducing LDL-C, good safety and convenience in administration, anti-PCSK9 monoclonal antibodies are drugs marking a major breakthrough in the field of lipid-lowering treatment.

There is still a demand for alternative anti-PCSK9 antibodies. Specifically, there is a demand for alternative anti-PCSK9 antibodies that have a high affinity for PCSK9, a reliable cell strain source, high stability, and high efficiency in reducing LDL-C. More specifically, there is a demand for alternative anti-PCSK9 antibodies that efficiently reduce LDL-C and provide a long duration of effect (such as long-lasting inhibition for LDL-C level). Such antibodies will also preferably have physical and chemical properties that are favorable for development, manufacturing or preparation. At present, most of the monoclonal antibody drugs on the market are administrated by intravenous injection. However, subcutaneous injection has become a preferred mode of injection because patients with some certain chronic diseases hope to be self-medicated at home. Since the volume for subcutaneous injection is generally limited to 1.0 mL to 1.5 mL, the antibody preparation needs to have a high concentration (≥ 100 mg/mL) to meet the requirements for clinical dosage.

Formulations comprising anti-PCSK9 antibodies are already disclosed (WO 2012/154999 A1 (AMGEN INC [US]; CHAN JOYCE CHI YEE [US] ET AL.) 15 November 2012 (2012-11-15)). High-concentration antibody preparations, however, also face a great challenge of high viscosity, because high-viscosity preparations will bring great difficulty to the downstream process and subcutaneous administration of drugs with a syringe. Under high concentrations, proteins are often less stable and are likely to aggregate and granulate. Moreover, the instability of protein conformations will also lead to a significant change in chemical stability such as charge heterogeneity.

In addition, as biomacromolecules, monoclonal antibodies have a very complex structure. In the process of production, expressed antibody molecules will undergo various post-translational modification and degradation, such as N-terminus cyclization, glycosylation, deamidization, isomerization, oxidation, fragmentation and disulfide bond mispairing. These quality attributes may affect the safety and effectiveness of final products, so it is also very important to control the correctness and consistency of product quality.

There is a need in the art to provide a liquid preparation comprising an anti-PCSK9 antibody with low viscosity, stability and high concentration.

### SUMMARY

PCSK9 is an important and beneficial therapeutic target. The present invention provides a liquid antibody preparation comprising an anti-PCSK9 antibody or a fragment thereof (such as the anti-PCSK9 antibody defined herein). The following embodiments are presented for illustrative purposes; the invention for which protection is sought is defined by the claims.

### I. Antibody Preparation Disclosed Herein

In one aspect, the present invention provides a liquid antibody preparation, which comprises:
(i) an anti-PCSK9 antibody or a fragment thereof (such as an antigen-binding fragment);
(ii) a buffer;
(iii) a viscosity inhibitor; and
(iv) a surfactant.

In one embodiment, the concentration of the anti-PCSK9 antibody or the fragment thereof in the liquid antibody preparation disclosed herein is about 50 mg/mL to about 200 mg/mL. In another embodiment, the concentration of the anti-PCSK9 antibody or the fragment thereof in the liquid antibody preparation disclosed herein is about 100 mg/mL to about 200 mg/mL. In another embodiment, the concentration of the anti-PCSK9 antibody or the fragment thereof in the liquid antibody preparation disclosed herein is about 100 mg/mL. In another embodiment, the concentration of the anti-PCSK9 antibody or the fragment thereof in the liquid antibody preparation disclosed herein is about 125 mg/mL. In another embodiment, the concentration of the anti-PCSK9 antibody or the fragment thereof in the liquid antibody preparation disclosed herein is about 150 mg/mL. In another embodiment, the concentration of the anti-PCSK9 antibody or the fragment thereof in the liquid antibody preparation disclosed herein is about 175 mg/mL. In another embodiment, the concentration of the anti-PCSK9 antibody or the fragment thereof in the liquid antibody preparation disclosed herein is about 200 mg/mL.

In one embodiment, the anti-PCSK9 antibody is any antibody binding to PCSK9 proteins (such as human PCSK9), such as a polyclonal antibody, a monoclonal antibody or a combination of the two. Preferably, in one embodiment, the anti-PCSK9 antibody is a monoclonal antibody. In one embodiment, the anti-PCSK9 antibody or the fragment thereof is the anti-PCSK9 antibody or the fragment thereof defined herein.

In one embodiment, the concentration of the buffer in the liquid antibody preparation disclosed herein is about 0.01 mg/mL to 50 mg/mL. In one embodiment, the concentration of the buffer in the liquid antibody preparation disclosed herein is about 0.1 mg/mL to 50 mg/mL. In one embodiment, the concentration of the buffer in the liquid antibody preparation disclosed herein is about 0.2 mg/mL to 10 mg/mL. In one embodiment, the concentration of the buffer in the liquid antibody preparation disclosed herein is about 0.5 mg/mL to 2.5 mg/mL. In one embodiment, the concentration of the buffer in the liquid antibody preparation disclosed herein is about 1.0 mg/mL to 2.0 mg/mL. In one embodiment, the concentration of the buffer in the liquid antibody preparation disclosed herein is about 1.5 mg/mL.

In one embodiment, the buffer is selected from histidine, glutamate, phosphate, acetate, citrate, and tris(hydroxymethyl)aminomethane. In one embodiment, the buffer is histidine.

In one embodiment, the concentration of the viscosity inhibitor in the liquid antibody preparation disclosed herein is about 10 mmol/L to 1000 mmol/L. In one embodiment, the concentration of the viscosity inhibitor in the liquid antibody preparation disclosed herein is about 20 mmol/L to 500 mmol/L. In one embodiment, the concentration of the viscosity inhibitor in the liquid antibody preparation disclosed herein is about 50 mmol/L to 300 mmol/L. In one embodiment, the concentration of the viscosity inhibitor in the liquid antibody preparation disclosed herein is about 50 mmol/L to 200 mmol/L.

In one embodiment, the viscosity inhibitor is selected from sugar alcohols (such as sorbitol), arginine, arginine hydrochloride, sodium thiocyanate, ammonium thiocyanate, ammonium sulfate, ammonium chloride, calcium chloride, zinc chloride, sodium acetate, and combinations thereof. In one embodiment, the viscosity inhibitor is sorbitol. In one embodiment, the viscosity inhibitor is sorbitol with a concentration of about 1% to 6% (w/v). In one embodiment, the viscosity inhibitor is sorbitol with a concentration of about 1% to 6% (w/v), and the liquid preparation does not comprise other viscosity inhibitors. In one embodiment, the viscosity inhibitor is a combination of sorbitol and arginine or arginine salt (preferably arginine hydrochloride). In one embodiment, the viscosity inhibitor is a combination of sorbitol and arginine or arginine salt (preferably arginine hydrochloride), wherein the concentration of sorbitol is about 1% to 6% (w/v), preferably about 2% to 4% (w/v), and more preferably about 3% (w/v), and the concentration of arginine or arginine salt (preferably arginine hydrochloride) is about 50 mmol/L to 180 mmol/L, preferably about 70 mmol/L to 150 mmol/L, and more preferably about 90 mmol/L. In one embodiment, the viscosity inhibitor is a combination of sorbitol and arginine or arginine salt (preferably arginine hydrochloride), wherein the concentration of sorbitol is about 1% to 6% (w/v), preferably about 2% to 4% (w/v), and more preferably about 3% (w/v), and the concentration of arginine or arginine salt is about 50 mmol/L to 180 mmol/L, preferably about 70 mmol/L to 150 mmol/L, and more preferably about 90 mmol/L, and the liquid preparation does not comprise other viscosity inhibitors. In one embodiment, the concentration of the surfactant is about 0.01 mg/mL to 5 mg/mL. In one embodiment, the concentration of the surfactant is about 0.05 mg/mL to 1 mg/mL. In one embodiment, the concentration of the surfactant is about 0.1 mg/mL to 0.5 mg/mL. In one embodiment, the concentration of the surfactant is about 0.3 mg/mL.

In one embodiment, the surfactant is a nonionic surfactant. In one embodiment, the surfactant is, for example, pluronics, polysorbate-80, polysorbate-60, polysorbate-40, or polysorbate-20.

In one embodiment, the liquid preparation disclosed herein comprises a solvent, and preferably, the solvent is selected from water for injection, organic solvents for injection (including but not limited to oil for injection, ethanol, and propylene glycol), and combinations thereof.

In one embodiment, the pH of the liquid preparation is about 5.0 to 6.0. In one embodiment, the pH of the liquid preparation is about 5.2 to 5.8. In one embodiment, the pH of the liquid preparation is about 5.4 to 5.6. In one embodiment, the pH of the liquid preparation is about 5.5.

In one embodiment, the viscosity of the liquid preparation at 25 °C is about 1.0 to 20 centipoises. In one embodiment, the viscosity of the liquid preparation at 25 °C is about 1.0 to 10 centipoises. In one embodiment, the viscosity of the liquid preparation at 25 °C is about 1.0, 2.0, 3.0, 4.0, 5.0, 6.0, 7.0, 8.0, 9.0, or 10.0 centipoises. In one embodiment, the viscosity of the liquid preparation at 25 °C is about 5.0 to 7.0 centipoises. In one embodiment, the viscosity of the liquid preparation at 25 °C is about 5.2 centipoises. In one embodiment, the viscosity of the liquid preparation at 25 °C is about 6.0 centipoises.

In one embodiment, the liquid preparation is a pharmaceutical preparation, preferably an injection, more preferably a subcutaneous injection.

In one preferred embodiment, the liquid antibody preparation disclosed herein comprises:
(i) about 100 mg/mL to 200 mg/mL of an anti-PCSK9 antibody or a fragment thereof;
(ii) about 0.2 mg/mL to 10 mg/mL of histidine;
(iii) about 1% to 6% (w/v) of sorbitol; and
(iv) about 0.05 mg/mL to 1 mg/mL of polysorbate-80 or polysorbate-20;
wherein the pH of the liquid preparation is about 5.0 to 6.0.

In one more preferred embodiment, the liquid antibody preparation disclosed herein also comprises water for injection.

In one preferred embodiment, the liquid antibody preparation disclosed herein comprises:
(i) about 100 mg/mL to 200 mg/mL of an anti-PCSK9 antibody or a fragment thereof;
(ii) about 0.2 mg/mL to 10 mg/mL of histidine;
(iii) about 1% to 6% of sorbitol and about 50 mmol/L to 180 mmol/L of arginine or arginine salt; and
(iv) about 0.05 mg/mL to 1 mg/mL of polysorbate-80 or polysorbate-20;
wherein the pH of the liquid preparation is about 5.0 to 6.0.

In one more preferred embodiment, the liquid antibody preparation disclosed herein also comprises water for injection.

In one embodiment, after the liquid preparation comprising an anti-PCSK9 antibody disclosed herein is stored at about -80 °C to about 45 °C, such as -80 °C, about -30 °C, about -20 °C, about 0 °C, about 5 °C, about 25 °C, about 35 °C, about 37 °C, about 42 °C or about 45 °C, for at least 14 days, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer, the purity of the anti-PCSK9 antibody or a fragment thereof decreases by no more than 10%, for example, no more than 5%, 4%, 3%, 2%, 1%, 0.5%, or 0.1%, as detected by SEC-HPLC.

In one embodiment, after the liquid preparation comprising an anti-PCSK9 antibody disclosed herein is stored at about -80 °C to about 45 °C, such as -80 °C, about -30 °C, about -20 °C, about 0 °C, about 5 °C, about 25 °C, about 35 °C, about 37 °C, about 42 °C or about 45 °C, for at least 14 days, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer, the purity of the anti-PCSK9 antibody or a fragment thereof decreases by no more than 10%, for example, no more than 5%, 4%, 3%, 2%, 1%, 0.5%, or 0.1%, as detected by non-reduced CE-SDS.

In one embodiment, after the liquid preparation comprising an anti-PCSK9 antibody disclosed herein is stored at about -80 °C to about 45 °C, such as -80 °C, about -30 °C, about -20 °C, about 0 °C, about 5 °C, about 25 °C, about 35 °C, about 37 °C, about 42 °C, or about 45 °C, for at least 14 days, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer, the charge heterogeneity of the anti-PCSK9 antibody or a fragment thereof changes by no more than 10%, for example, no more than 5%, 4%, 3%, 2%, 1%, 0.5%, or 0.1%, as detected by CEX-HPLC.

The preparation disclosed herein has a high antibody concentration, a low viscosity suitable for administration (particularly subcutaneous administration), and a high physical and chemical stability, and is less prone to aggregate and granulate and exhibit heterogeneity change when stored. These advantages are very beneficial to the production of complete, effective, and highly consistent clinical drugs.

The inventors surprisingly found that sorbitol can be used as a viscosity inhibitor for the liquid preparation comprising an anti-PCSK9 antibody, in which case a liquid preparation comprising an anti-PCSK9 antibody or a fragment thereof having low viscosity and high concentration can be obtained without adding other viscosity inhibitors, and the liquid preparation has high stability. In addition, the inventors surprisingly found that a combination of sorbitol and arginine as a viscosity inhibitor can better reduce the viscosity of the liquid preparation comprising an anti-PCSK9 antibody and enable a high-concentration liquid preparation comprising an anti-PCSK9 antibody or a fragment thereof to have lower viscosity suitable for subcutaneous injection lower viscosity suitable for subcutaneous injection, and the liquid preparation comprising an anti-PCSK9 antibody has higher stability (see examples 10-12).

Therefore, in another aspect, the present invention provides use of sugar alcohol as a viscosity inhibitor for the liquid preparation comprising an anti-PCSK9 antibody.

In one embodiment, the concentration of the sugar alcohol in the liquid preparation comprising an anti-PCSK9 antibody is about 1% to 6% (w/v), preferably about 2% to 4% (w/v), and more preferably about 3% (w/v). In one embodiment, the sugar alcohol is preferably sorbitol.

In another aspect, the present invention provides use of sugar alcohol as a viscosity inhibitor in preparing the liquid preparation comprising an anti-PCSK9 antibody.

In one embodiment, the present invention provides use of sugar alcohol as the only viscosity inhibitor in preparing the liquid preparation comprising an anti-PCSK9 antibody.

In one embodiment, the concentration of the sugar alcohol in the liquid preparation comprising an anti-PCSK9 antibody is about 1% to 6% (w/v), preferably about 2% to 4% (w/v), and more preferably about 3% (w/v). In one embodiment, the sugar alcohol is preferably sorbitol.

In another aspect, the present invention provides use of a combination of sugar alcohol and arginine (or arginine salt, preferably arginine hydrochloride) as a viscosity inhibitor for the liquid preparation comprising an anti-PCSK9 antibody.

In one embodiment, the concentration of the sugar alcohol in the liquid preparation comprising an anti-PCSK9 antibody is about 1% to 6% (w/v), preferably about 2% to 4% (w/v), and more preferably about 3% (w/v); and the concentration of arginine or arginine salt (preferably arginine hydrochloride) is about 50 mmol/L to 180 mmol/L, preferably about 70 mmol/L to 150 mmol/L, and more preferably about 90 mmol/L. In one embodiment, the sugar alcohol is preferably sorbitol. In another aspect, the present invention provides use of a combination of sugar alcohol and arginine (or arginine salt, preferably arginine hydrochloride) as a viscosity inhibitor in preparing the liquid preparation comprising an anti-PCSK9 antibody.

In one embodiment, the present invention provides use of a combination of sugar alcohol and arginine (or arginine salt, preferably arginine hydrochloride) as the only viscosity inhibitor in preparing the liquid preparation comprising an anti-PCSK9 antibody.

In one embodiment, the concentration of the sugar alcohol in the liquid preparation comprising an anti-PCSK9 antibody is about 1% to 6% (w/v), preferably about 2% to 4% (w/v), and more preferably about 3% (w/v); and the concentration of arginine or arginine salt (preferably arginine hydrochloride) is about 50 mmol/L to 180 mmol/L, preferably about 70 mmol/L to 150 mmol/L, and more preferably about 90 mmol/L. In one embodiment, the sugar alcohol is preferably sorbitol. In another aspect, the present invention provides a solid preparation obtained by lyophilizing the aforementioned liquid preparation. Before use, the solid preparation can be reconstituted in a suitable solvent to give the liquid preparation disclosed herein.

In another aspect, the present invention provides a method for preparing the liquid preparation disclosed herein, which comprises the following steps:
a. providing an anti-PCSK9 antibody or a fragment thereof;
b. adding a buffer and a viscosity inhibitor;
c. adding a surfactant; and
d. optionally conducting sterile filtration to give the liquid preparation disclosed herein.

In one embodiment, the anti-PCSK9 antibody or the fragment thereof in step a is a separated and purified anti-PCSK9 antibody.

In one embodiment, the anti-PCSK9 antibody or the fragment thereof in step a is provided in the form of a solution, preferably an aqueous solution.

In one embodiment, before step b, the anti-PCSK9 antibody or the fragment thereof is concentrated by being centrifuged using an ultrafiltration centrifuge tube having a molecular weight cutoff of, for example, about 30 KD.

In one embodiment, the buffer and the viscosity inhibitor in step b are provided in the form of a solution, preferably an aqueous solution.

In one embodiment, after step b, the anti-PCSK9 antibody or the fragment thereof is concentrated by ultrafiltration and centrifugation and then diluted with the solutions, preferably aqueous solutions, of the buffer and the viscosity inhibitor, and this is repeated until the replacement is completed.

In one embodiment, before step c, the concentration of the anti-PCSK9 antibody or the fragment thereof is adjusted.

The anti-PCSK9 antibody or the fragment thereof, the buffer, the viscosity inhibitor, and the surfactant are as described herein, and doses and/or concentrations thereof are as described for, or can be appropriately determined by, the liquid preparation disclosed herein.

In one embodiment, the method for preparing the liquid preparation disclosed herein comprises the following steps:
i. providing a separated and purified anti-PCSK9 antibody, optionally adding the antibody to an ultrafiltration centrifuge tube with a molecular weight cutoff of about 30 KD, and concentrating the antibody by centrifugation;
ii. diluting the anti-PCSK9 antibody with solutions, preferably aqueous solutions, of a buffer and a viscosity inhibitor (preferably, the variety, concentration, and solution pH of the buffer and the viscosity inhibitor are as defined above and as defined for the liquid preparation disclosed herein in the definition section, and more preferably, the buffer is histidine and the viscosity inhibitor is arginine and/or sorbitol) and then concentrating, and repeating this until the replacement is completed;
iii. adjusting the concentration of replaced protein to a concentration defined for the liquid preparation disclosed herein;
iv. adding a surfactant or a solution thereof, preferably an aqueous solution, and adjusting the final concentration of the surfactant to a concentration defined for the liquid preparation disclosed herein;
v. optionally aseptically filtering the solution in step iv; and
vi. optionally aliquoting the solution into vials and capping the vials with rubber stoppers and aluminum-plastic caps to give the product.

In the embodiment, the anti-PCSK9 antibody is the anti-PCSK9 antibody defined herein.

### II. Anti-PCSK9 Antibody Disclosed Herein

In some embodiments, an anti-PCSK9 antibody or an antibody fragment (preferably an antigen-binding fragment) binding to PCSK9 or a fragment thereof (preferably human PCSK9 protein) is provided.

In one aspect of the present invention, an anti-PCSK9 antibody and a fragment thereof (such as an antigen-binding fragment) are provided herein. In some embodiments, the anti-PCSK9 antibody inhibits or blocks the activity of PCSK9. In some embodiments, the anti-PCSK9 antibody disclosed herein has one or more of the following properties:
(1) binding to PCSK9 (preferably human PCSK9 protein), preferably with an equilibrium dissociation constant (K_{D}) less than or equal to about 1 µM, about 100 nM, about 10 nM, about 1 nM, about 0.1 nM, about 0.01 nM, or about 0.001 nM (*e.g.,* 10⁻⁸ M or less, *e.g.,* 10⁻⁸ M to 10⁻¹³ M, *e.g.,* 10⁻⁹ M to 10 ⁻¹³ M);
(2) blocking the binding of PCSK9 (such as human PCSK9) to LDLR (such as human LDLR);
(3) enhancing the capability of restoring LDLR and/or inhibiting the decrease of LDL-C uptake induced by PCSK9, thus increasing the uptake of LDL-C by cells (such as hepatocytes); preferably, the antibody concentration is about 10 nM, 15 nM, 20 nM, 30 nM, 35 nM, 40 nM, 45 nM, 50 nM, 55 nM, 60 nM, 65 nM, 70 nM, 75 nM, 80 nM, 85 nM, 90 nM, 95 nM, 100 nM or more, *e.g.,* about 10-100 nM and 15-70 nM; preferably, the capability of the antibody in increasing the uptake of LDL-C by cells is at least 2 times higher than that of a control antibody; and preferably, the control antibody is IgG or an antibody known in the art, such as Alirocumab, Evolocumab or Lodelcizumab;
(4) effectively preventing the internalization of LDLR in cells;
(5) (dose-dependently) lowering the level of LDL-C and/or the level of total cholesterol (TC) in serum;
(6) significantly lowering the level of LDL-C in serum over a long period of time, for example, a duration of response of more than 15, 16, 17, 18, 19, 20, or 21 days.

In some embodiments, the anti-PCSK9 antibody disclosed herein or the antigen-binding fragment thereof comprises a heavy chain variable region (VH), wherein the VH comprises:
(i) three complementarity determining regions (CDRs) contained in a VH of any of the antibodies listed in Table II, or
(ii) relative to the sequence of (i), a sequence comprising a total of at least one and no more than 5, 4, 3, 2, or 1 amino acid alteration (preferably amino acid replacement, and more preferably conservative replacement) in the three CDRs.

In some embodiments, the anti-PCSK9 antibody or the antigen-binding fragment thereof disclosed herein comprises a light chain variable region (VL), wherein the VL comprises:
(i) three complementarity determining regions (CDRs) contained in a VL set forth in SEQ ID NO: 24; or
(ii) relative to the sequence of (i), a sequence comprising a total of at least one and no more than 5, 4, 3, 2, or 1 amino acid alteration (preferably amino acid replacement, and more preferably conservative replacement) in the three CDRs.

In some embodiments, the anti-PCSK9 antibody or the antigen-binding fragment thereof disclosed herein comprises a heavy chain variable region VH and a light chain variable region VL, wherein,
(a) the VH comprises:
   (i) three complementarity determining regions (CDRs) contained in a VH of any of the antibodies listed in Table II, or
   (ii) relative to the sequence of (i), a sequence comprising a total of at least one and no more than 5, 4, 3, 2, or 1 amino acid alteration (preferably amino acid replacement, and more preferably conservative replacement) in the three CDRs; and/or
(b) the VL comprises:
   (i) three complementarity determining regions (CDRs) contained in a VL set forth in SEQ ID NO: 24; or
   (ii) relative to the sequence of (i), a sequence comprising a total of at least one and no more than 5, 4, 3, 2, or 1 amino acid alteration (preferably amino acid replacement, and more preferably conservative replacement) in the three CDRs.

In a preferred embodiment, the VH comprises or consists of an amino acid sequence selected from SEQ ID NOs: 23, 25, 26, 27, 28, 29, 30, 31, 32, and 33.

In a preferred embodiment, the VL comprises or consists of an amino acid sequence set forth in SEQ ID NO: 24.

In a preferred embodiment, the anti-PCSK9 antibody or the antigen-binding fragment thereof disclosed herein comprises:
(a) three complementarity determining regions of a heavy chain variable region (HCDRs) set forth in SEQ ID NO: 23, 25, 26, 27, 28, 29, 30, 31, 32, or 33, and/or
(b) three complementarity determining regions of a light chain variable region (LCDRs) set forth in SEQ ID NO:24.

In a preferred embodiment, the anti-PCSK9 antibody or the antigen-binding fragment thereof disclosed herein comprises:
(a) three complementarity determining regions of a heavy chain variable region (HCDRs) set forth in SEQ ID NO: 23, 25, 26, 27, 28, 29, 30, 31, 32, or 33, and
(b) three complementarity determining regions of a light chain variable region (LCDRs) set forth in SEQ ID NO:24.

In some embodiments, the anti-PCSK9 antibody or the antigen-binding fragment thereof disclosed herein comprises a heavy chain variable region (VH) and/or a light chain variable region (VL), wherein
(i) the VH comprises complementarity determining regions (CDRs) HCDR1, HCDR2, and HCDR3, wherein the HCDR1 comprises or consists of an amino acid sequence selected from SEQ ID NOs: 1, 7, 8, 9, 10, 11, 12, 13, and 20, or the HCDR1 comprises an amino acid sequence having one, two, or three alterations (preferably amino acid replacements, and more preferably conservative replacements) compared with the amino acid sequence selected from SEQ ID NOs: 1, 7, 8, 9, 10, 11, 12, 13, and 20; the HCDR2 comprises or consists of an amino acid sequence selected from SEQ ID NOs: 2, 14, 15, 16, 17, and 21, or the HCDR2 comprises an amino acid sequence having one, two, or three alterations (preferably amino acid replacements, and more preferably conservative replacements) compared with the amino acid sequence selected from SEQ ID NOs: 2, 14, 15, 16, 17, and 21; and the HCDR3 comprises or consists of an amino acid sequence selected from SEQ ID NOs: 3, 18, 19, and 22, or the HCDR3 comprises an amino acid sequence having one, two, or three alterations (preferably amino acid replacements, and more preferably conservative replacements) compared with the amino acid sequence selected from SEQ ID NOs: 3, 18, 19, and 22;
   and/or
(ii) the VL comprises complementarity determining regions (CDRs) LCDR1, LCDR2, and LCDR3, wherein the LCDR1 comprises or consists of an amino acid sequence set forth in SEQ ID NO: 4, or the LCDR1 comprises an amino acid sequence having one, two, or three alterations (preferably amino acid replacements, and more preferably conservative replacements) compared with the amino acid sequence set forth in SEQ ID NO: 4; the LCDR2 comprises or consists of an amino acid sequence set forth in SEQ ID NO: 5, or the LCDR2 comprises an amino acid sequence having one, two, or three alterations (preferably amino acid replacements, and more preferably conservative replacements) compared with the amino acid sequence set forth in SEQ ID NO: 5; and the LCDR3 comprises or consists of an amino acid sequence set forth in SEQ ID NO: 6, or the LCDR3 comprises an amino acid sequence having one, two, or three alterations (preferably amino acid replacements, and more preferably conservative replacements) compared with the amino acid sequence set forth in SEQ ID NO: 6.

In a preferred embodiment, the present invention provides an anti-PCSK9 antibody or an antigen-binding fragment thereof comprising a heavy chain variable region (VH) and/or a light chain variable region (VL), wherein
(a) the VH comprises:
   (i) a combination of the HCDR1, HCDR2, and HCDR3 shown in Table I; or
   (ii) a variant of the HCDR combination in (i), comprising a total of at least one and no more than 5, 4, 3, 2, or 1 amino acid alteration (preferably amino acid replacement, and more preferably conservative replacement) in the three CDRs;
      and/or
(ii) the VL comprises:
   (i) a combination of an LCDR1, an LCDR2, and an LCDR3 respectively comprising or consisting of amino acid sequences set forth in SEQ ID NOs: 4, 5, and 6; or
   (ii) a variant of the LCDR combination of (i), comprising a total of at least one and no more than 5, 4, 3, 2, or 1 amino acid alteration (preferably amino acid replacement, and more preferably conservative replacement) in the three CDRs.

In a preferred embodiment, the present invention provides an anti-PCSK9 antibody or an antigen-binding fragment thereof, comprising a heavy chain variable region (VH) and/or a light chain variable region (VL), wherein the VH comprises complementarity determining regions (CDRs) HCDR1, HCDR2, and HCDR3, and the VL comprises (CDRs) LCDR1, LCDR2, and LCDR3; and combinations of the HCDR1, HCDR2, HCDR3, LCDR1, LCDR2, and LCDR3 contained in the antibody or the antigen-binding fragment thereof are shown in the following table (Table I):

**Table I: Exemplary combinations of the HCDR1, HCDR2, HCDR3, LCDR1, LCDR2, and LCDR3 in the antibody or the antigen-binding fragment thereof disclosed herein**

| Combinations | HCDR1, which comprises or consists of an amino acid sequence set forth in the following SEQ ID Nos | HCDR2, which comprises or consists of an amino acid sequence set forth in the following SEQ ID Nos | HCDR3, which comprises or consists of an amino acid sequence set forth in the following SEQ ID Nos | LCDR1, which comprises or consists of an amino acid sequence set forth in the following SEQ ID Nos | LCDR2, which comprises or consists of an amino acid sequence set forth in the following SEQ ID Nos | LCDR3, which comprises or consists of an amino acid sequence set forth in the following SEQ ID Nos |
|---|---|---|---|---|---|---|
| (1) | SEQ ID NO:1 | SEQ ID NOT | SEQ ID NO:3 | SEQ ID NO: 4 | SEQ ID NO:5 | SEQ ID NO:6 |
| (2) | SEQ ID NO:1 | SEQ ID NOT | SEQ ID NO:18 | SEQ ID NO: 4 | SEQ ID NO:5 | SEQ ID NO:6 |
| (3) | SEQ ID NO:1 | SEQ ID NO:14 | SEQ ID NO:19 | SEQ ID NO: 4 | SEQ ID NO:5 | SEQ ID NO:6 |
| (4) | SEQ ID NO:7 | SEQ ID NO:15 | SEQ ID NO:18 | SEQ ID NO: 4 | SEQ ID NO:5 | SEQ ID NO:6 |
| (5) | SEQ ID NO:8 | SEQ ID NO:16 | SEQ ID NO:19 | SEQ ID NO: 4 | SEQ ID NO:5 | SEQ ID NO:6 |
| (6) | SEQ ID NO:7 | SEQ ID NO:17 | SEQ ID NO:19 | SEQ ID NO: 4 | SEQ ID NO:5 | SEQ ID NO:6 |
| (7) | SEQ ID NO:10 | SEQ ID NO:17 | SEQ ID NO:19 | SEQ ID NO: 4 | SEQ ID NO:5 | SEQ ID NO:6 |
| (8) | SEQ ID NO:11 | SEQ ID NO:17 | SEQ ID NO:18 | SEQ ID NO: 4 | SEQ ID NO:5 | SEQ ID NO:6 |
| (9) | SEQ ID NO:12 | SEQ ID NO:17 | SEQ ID NO:18 | SEQ ID NO: 4 | SEQ ID NO:5 | SEQ ID NO:6 |
| (10) | SEQ ID NO:13 | SEQ ID NO:17 | SEQ ID NO:18 | SEQ ID NO: 4 | SEQ ID NO:5 | SEQ ID NO:6 |
| (11) | SEQ ID NO:20 | SEQ ID NO:21 | SEQ ID NO:22 | SEQ ID NO: 4 | SEQ ID NO:5 | SEQ ID NO:6 |

In a preferred embodiment, the present invention provides an anti-PCSK9 antibody or an antigen-binding fragment thereof comprising a combination of the HCDR1, HCDR2, HCDR3, LCDR1, LCDR2, and LCDR3 shown in Table I.

In some embodiments, the anti-PCSK9 antibody or the antigen-binding fragment thereof disclosed herein comprises a heavy chain variable region (VH) and/or a light chain variable region (VL), wherein,
(a) the heavy chain variable region VH
   (i) comprises or consists of an amino acid sequence having at least 90%, 91%, 92%, 93%, 94%, 95%, 96%, 97%, 98%, or 99% identity to an amino acid sequence selected from SEQ ID NOs: 23, 25, 26, 27, 28, 29, 30, 31, 32, and 33; or
   (ii) comprises or consists of an amino acid sequence selected from SEQ ID NO: 23, 25, 26, 27, 28, 29, 30, 31, 32, and 33; or
   (iii) comprises an amino acid sequence having one or more (preferably no more than 10, and more preferably no more than 5, 4, 3, 2, or 1) amino acid alterations (preferably amino acid replacements, and more preferably conservative amino acid replacements) compared with an amino acid sequence selected from SEQ ID NOs: 23, 25, 26, 27, 28, 29, 30, 31, 32, and 33, wherein preferably, the amino acid alterations do not occur in the CDRs;
      and/or
(b) the light chain variable region VL
   (i) comprises or consists of an amino acid sequence having at least 90%, 91%, 92%, 93%, 94%, 95%, 96%, 97%, 98%, or 99% identity to an amino acid sequence set forth in SEQ ID NO: 24;
   (ii) comprises or consists of an amino acid sequence set forth in SEQ ID NO: 24; or
   (iii) comprises an amino acid sequence having one or more (preferably no more than 10, and more preferably no more than 5, 4, 3, 2, or 1) amino acid alterations (preferably amino acid replacements, and more preferably conservative amino acid replacements) compared with an amino acid sequence set forth in SEQ ID NO: 24, wherein preferably, the amino acid alterations do not occur in the CDRs.

In a preferred embodiment, the present invention provides an anti-PCSK9 antibody or an antigen-binding fragment thereof comprising a heavy chain variable region (VH) and a light chain variable region (VL), wherein combinations of the heavy chain variable region (VH) and the light chain variable region (VL) contained in the antibody or the antigen-binding fragment thereof are shown in the following table (Table II):

**Table II: Exemplary combinations of the heavy chain variable region VH and light chain variable region VL in the antibody or the antigen-binding fragment thereof disclosed herein**

| Combinations | VH, which comprises or consists of an amino acid sequence set forth in the following SEQ ID NOs | VL, which comprises or consists of an amino acid sequence set forth in the following SEQ ID NOs |
|---|---|---|
| (1) | SEQ ID NO:23 | SEQ ID NO:24 |
| (2) | SEQ ID NO:25 | SEQ ID NO:24 |
| (3) | SEQ ID NO:26 | SEQ ID NO:24 |
| (4) | SEQ ID NO:27 | SEQ ID NO:24 |
| (5) | SEQ ID NO:28 | SEQ ID NO:24 |
| (6) | SEQ ID NO:29 | SEQ ID NO:24 |
| (7) | SEQ ID NO:30 | SEQ ID NO:24 |
| (8) | SEQ ID NO:31 | SEQ ID NO:24 |
| (9) | SEQ ID NO:32 | SEQ ID NO:24 |
| (10) | SEQ ID NO:33 | SEQ ID NO:24 |

In some embodiments, the anti-PCSK9 antibody or the antigen-binding fragment thereof disclosed herein comprises a heavy chain and/or a light chain, wherein
(a) the heavy chain
   (i) comprises or consists of an amino acid sequence having at least 85%, 90%, 91%, 92%, 93%, 94%, 95%, 96%, 97%, 98%, or 99% identity to an amino acid sequence selected from SEQ ID NOs: 34, 36, 37, 38, 39, 40, 41, 42, 43, and 44;
   (ii) comprises or consists of an amino acid sequence selected from SEQ ID NO: 34, 36, 37, 38, 39, 40, 41, 42, 43, and 44; or
   (iii) comprises an amino acid sequence having one or more (preferably no more than 20 or 10, and more preferably no more than 5, 4, 3, 2, or 1) amino acid alterations (preferably amino acid replacements, and more preferably conservative amino acid replacements) compared with an amino acid sequence selected from SEQ ID NOs: 34, 36, 37, 38, 39, 40, 41, 42, 43, and 44, wherein preferably, the amino acid alterations do not occur in the CDRs of the heavy chain, and more preferably, the amino acid alterations do not occur in the heavy chain variable region;
      and/or
(b) the light chain
   (i) comprises or consists of an amino acid sequence having at least 85%, 90%, 91%, 92%, 93%, 94%, 95%, 96%, 97%, 98%, or 99% identity to an amino acid sequence set forth in SEQ ID NO: 35;
   (ii) comprises or consists of an amino acid sequence set forth in SEQ ID NO: 35; or
   (iii) comprises an amino acid sequence having one or more (preferably no more than 20 or 10, and more preferably no more than 5, 4, 3, 2, or 1) amino acid alterations (preferably amino acid replacements, and more preferably conservative amino acid replacements) compared with an amino acid sequence set forth in SEQ ID NO: 35, wherein preferably, the amino acid alterations do not occur in the CDRs of the light chain, and more preferably, the amino acid alterations do not occur in the light chain variable region.

In a preferred embodiment, the present invention provides an anti-PCSK9 antibody or an antigen-binding fragment thereof comprising a heavy chain and a light chain, wherein combinations of the heavy chain and the light chain contained in the antibody or the antigen-binding fragment thereof are shown in the following table (Table III):

**Table III: Exemplary combinations of the heavy chain and light chain in the antibody or the antigen-binding fragment thereof disclosed herein**

| Combinations | Heavy chain, which comprises or consists of an amino acid sequence set forth in the following SEQ ID NOs | Light chain, which comprises or consists of an amino acid sequence set forth in the following SEQ ID NOs |
|---|---|---|
| (1) | SEQ ID NO:34 | SEQ ID NO:35 |
| (2) | SEQ ID NO:36 | SEQ ID NO:35 |
| (3) | SEQ ID NO:37 | SEQ ID NO:35 |
| (4) | SEQ ID NO:38 | SEQ ID NO:35 |
| (5) | SEQ ID NO:39 | SEQ ID NO:35 |
| (6) | SEQ ID NO:40 | SEQ ID NO:35 |
| (7) | SEQ ID NO:41 | SEQ ID NO:35 |
| (8) | SEQ ID NO:42 | SEQ ID NO:35 |
| (9) | SEQ ID NO:43 | SEQ ID NO:35 |
| (10) | SEQ ID NO:44 | SEQ ID NO:35 |

In some embodiments, the antibody disclosed herein also encompasses a variant of the amino acid sequence of an anti-PCSK9 antibody, as well as an antibody that binds to the same epitope as any of the antibodies described above.

In certain embodiments, an antibody or an antibody fragment (preferably an antigen-binding fragment) that binds to PCSK9 or a fragment thereof is provided, wherein the antibody binds to an epitope within the fragment of PCSK9. In certain embodiments, an antibody or an antibody fragment that binds to PCSK9 or a fragment thereof is provided, wherein the antibody binds to an epitope within an amino acid fragment comprising human PCSK9 amino acid sequence set forth in SEQ ID NO: 53.

In some embodiments, the heavy chain and/or light chain of the anti-PCSK9 antibody or the fragment thereof disclosed herein further comprises a signal peptide sequence, such as METDTLLLWVLLLWVPGSTG.

In one embodiment of the present invention, the amino acid alteration described herein includes amino acid replacement, insertion or deletion. Preferably, the amino acid alteration described herein is an amino acid replacement, preferably a conservative replacement.

In a preferred embodiment, the amino acid alteration described herein occurs in a region outside the CDR (e.g., in FR). More preferably, the amino acid alteration described herein occurs in a region outside the heavy chain variable region and/or outside the light chain variable region.

In some embodiments, the replacement is a conservative replacement. A conservative replacement refers to the replacement of an amino acid by another amino acid of the same class, e.g., the replacement of an acidic amino acid by another acidic amino acid, the replacement of a basic amino acid by another basic amino acid, or the replacement of a neutral amino acid by another neutral amino acid. Exemplary replacements are shown in Table IV below:

**Table IV**

| Primitive residue | Exemplary replacement | Preferred conservative amino acid replacement |
|---|---|---|
| Ala (A) | Val, Leu, Ile | Val |
| Arg (R) | Lys, Gln, Asn | Lys |
| Asn (N) | Gln, His, Asp, Lys, Arg | Gln |
| Asp (D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Asn, Gln, Lys, Arg | Arg |
| Ile (I) | Leu, Val, Met, Ala, Phe, Nle | Leu |
| Leu (L) | Nle, Ile, Val, Met, Ala, Phe | Ile |
| Lys (K) | Arg, Gln, Asn | Arg |
| Met (M) | Leu, Phe, Ile | Leu |
| Phe (F) | Trp, Leu, Val, Ile, Ala, Tyr | Tyr |
| Pro (P) | Ala | Ala |
| Ser (S) | Thr | Thr |
| Thr (T) | Val, Ser | Ser |
| Trp (W) | Tyr, Phe | Tyr |
| Tyr (Y) | Trp, Phe, Thr, Ser | Phe |
| Val (V) | Ile, Leu, Met, Phe, Ala, Nle | Leu |

In certain embodiments, the replacement occurs in the CDRs of the antibody. Generally, an obtained variant has modifications (*e.g.,* improvements) in certain biological properties (*e.g.,* increased affinity) relative to the parent antibody, and/or will substantially retain certain biological properties of the parent antibody. Exemplary replacement variants are affinity mature antibodies.

In certain embodiments, the antibody disclosed herein is altered to increase or decrease the extent to which the antibody is glycosylated. Addition or deletion of glycosylation sites to an antibody can be conveniently achieved by altering the amino acid sequence such that one or more glycosylation sites are created or removed. When the antibody comprises an Fc region, carbohydrate attached thereto can be altered. In some applications, modifications that remove undesired glycosylation sites may be useful, for example, removing fucose modules to enhance antibody-dependent cellular cytotoxicity (ADCC) function (see Shield, et al. (2002) JBC277:26733). In other applications, galactosidylation modification can be carried out to modify complement-dependent cytotoxicity (CDC).

In certain embodiments, one or more amino acid modifications can be introduced into an Fc region of the antibody disclosed herein, thereby producing an Fc region variant, such that, for example, the efficacy of the antibody in treating diseases is enhanced. The Fc region variant may comprise a human Fc region sequence (such as human IgG1, IgG2, IgG3, or IgG4 Fc region) comprising an amino acid modification (such as replacement) at one or more amino acid positions. For examples of the Fc variant, see U.S. Patent No. 7,332,581, U.S. Patent No. 6,737,056, U.S. Patent No. 6,737,056; WO 2004/056312 and Shields, et al., J. Biol. Chem. 9(2):6591-6604(2001), U.S. Patent No. 6,194,551, WO 99/51642 and Idusogie, et al., J. Immunol. 164:4178-4184(2000), U.S. Patent No. 7,371,826, Duncan & Winter, Nature 322:738-40(1988); U.S. Patent No. 5,648,260; U.S. Patent No. 5,624,821; and WO 94/29351.

In certain embodiments, antibodies modified by cysteine engineering may need to be produced, such as "sulfo-MAb", wherein one or more residues of the antibodies are replaced by cysteine residues. A cysteine-modified antibody can be produced as described, for example, in U.S. Patent No. 7,521,541.

In certain embodiments, the antibody disclosed herein can be further modified to comprise other non-protein portions known in the art and readily available. Suitable portions for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone), polyethylene glycol, propylene glycol homopolymer, polypropylene oxide/ethylene oxide copolymer, polyoxyethylated polyol (such as glycerol), polyvinyl alcohol, and mixtures thereof.

In some embodiments, the anti-PCSK9 antibody or the antigen-binding fragment thereof disclosed herein has one or more of the following properties:
(i) exhibiting binding affinity and/or specificity for PCSK9 (such as human PCSK9, preferably having an amino acid sequence set forth in SEQ ID NO: 53) identical with or similar to that of any one of the antibodies listed in Table C;
(ii) inhibiting (*e.g.,* competitively inhibiting) binding of any one of the antibodies listed in Table C to PCSK9 (such as human PCSK9, preferably having the amino acid sequence set forth in SEQ ID NO: 53);
(iii) binding to an identical or overlapping epitope as any one of the antibodies listed in Table C;
(iv) competing with any one of the antibodies listed in Table C for binding to PCSK9 (such as human PCSK9, preferably having the amino acid sequence set forth in SEQ ID NO: 53);
(v) having one or more biological properties of any one of the antibodies listed in Table C.

In some embodiments, the anti-PCSK9 antibody disclosed herein is an IgG1 antibody, an IgG2 antibody, or an IgG4 antibody.

In some embodiments, the anti-PCSK9 antibody disclosed herein is a monoclonal antibody. In some embodiments, the anti-PCSK9 antibody disclosed herein is humanized. In some embodiments, the anti-PCSK9 antibody disclosed herein is a human antibody. In some embodiments, at least a portion of the framework sequence of the anti-PCSK9 antibody disclosed herein is a human consensus framework sequence. In one embodiment, the anti-PCSK9 antibody disclosed herein also encompasses an antibody fragment thereof. Examples of the antibody fragment include, but are not limited to, Fv, Fab, Fab', Fab'-SH, F(ab')₂, a diabody, a linear antibody, a single-chain antibody molecule (*e.g.,* scFv), and a multispecific antibody formed from the antibody fragment.

An antibody is digested by papain to produce two identical antigen-binding fragments called "Fab" fragments, each having a single antigen-binding site, and a residual "Fc" fragment, the name of which reflects its ability to crystallize easily. An F(ab')₂ fragment having two antigen-binding sites and still being capable of being cross-linked with an antigen is produced by treatment of pepsin.

In some embodiments, the anti-PCSK9 antibody disclosed herein is a humanized antibody. Different methods for humanizing antibodies are known to those skilled, as summarized by Almagro & Fransson, (Almagro J.C. and Fransson J (2008) Frontiers in Bioscience 13:1619-1633). Almagro & Fransson distinguish between rational approach and empirical approach. The rational approach is characterized by generating a few engineered antibody variants and assessing their binding or any other property of interest. If the designed variants do not produce expected results, a new round of design and evaluation will be launched. The rational approach includes CDR grafting, resurfacing, superhumanization, and human string content optimization. In contrast, the empirical approach is based on generating large humanized variant libraries, and selects the best clones using enrichment techniques or high-throughput screening. Thus, the empirical approach depends on a reliable selection and/or screening system capable of searching for a large number of antibody variants. *In vitro* display technologies such as phage display and ribosome display meet these requirements and are well known to those skilled. The empirical approach includes FR library construction, guided selection, framework-shuffling, and humaneering.

In some embodiments, the anti-PCSK9 antibody disclosed herein is a human antibody. The human antibody can be prepared using a variety of techniques known in the art. The human antibody is generally described in van Dijk and van de Winkel, Curr. Opin. Pharmacol 5:368-74(2001) and Lonberg, Curr. Opin. Immunol 20:450-459(2008*).*

In some embodiments, the anti-PCSK9 antibody disclosed herein is a chimeric antibody.

The antibody disclosed herein can be isolated by screening antibodies having desired activity in a combinatorial library. For example, various methods for generating phage display libraries and screening antibodies with desired binding characteristics in the libraries are known in the art. The methods are reviewed in, for example, Hoogenboom et al., Methods in Molecular Biology 178:1-37 (edited by O' Brien et al., Human Press, Totowa, NJ, 2001), and further described in, for example, McCafferty et al., Nature 348:552-554; Clackso et al., Nature 352:624-628 (1991); Marks et al., J.Mol.Biol. 222:581-597(1992); Marks and Bradbury, Methods in Molecular Biology 248:161-175 (edited by Lo, Human Press, Totowa, NJ, 2003); Sidhu et al., J.Mol.Biol. 338(2):299-310(2004); Lee et al., J.Mol.Biol 340(5):1073-1093(2004); Fellouse, Proc.Natl.Acad.Sci. USA 101 (34):12467-12472(2004); and Lee et al., J.Immunol.Methods 284(1-2):119-132(2004).

In some embodiments, the present invention also encompasses an anti-PCSK9 monoclonal antibody ("conjugate or immunoconjugate") conjugated to other substances, such as a detectable label (such as fluorescent dye, enzyme, substrate, bioluminescent substance, radioactive substance, and chemiluminescent portion), a cytotoxic agent or an immunosuppressant. The cytotoxic agent includes any agent that is harmful to cells. Examples of the cytotoxic agent (such as a chemotherapeutic agent) suitable for forming the immunoconjugate are known in the art, see, for example, WO 05/103081.

In some embodiments, the antibody disclosed herein may be monospecific, bispecific, or multispecific. The multispecific monoclonal antibody may be specific to different epitopes of a target polypeptide or may comprise antigen-binding domains specific to more than one target polypeptide. See, for example, Tutt *et al.* (1991) *J.Immunol.*147:60-69. The anti-PCSK9 monoclonal antibody may be linked to or co-expressed with another functional molecule (such as another peptide or protein). For example, the antibody or a fragment thereof may be functionally linked to one or more other molecules, such as another antibody or antibody fragment (for example, by chemical coupling, genetic fusion, non-covalent association, or other methods), to produce a bispecific or multispecific antibody having a second or more binding specificities.

In some embodiments, the antibody disclosed herein binds to human PCSK9 protein, such as human PCSK9 protein set forth in SEQ ID NO: 53.

### III. Therapeutic Method and Use of the Antibody Disclosed Herein or Preparation Thereof

Any references in the description to methods of treatment refer to the compounds, pharmaceutical compositions and medicaments of the present invention for use in a method for treatment of the human body by therapy.

In another aspect, the present invention relates to a method for inhibiting the binding of PCSK9 to LDL-receptor (LDLR) in a subject, wherein the method comprises administering to the subject an effective amount of any of the anti-PCSK9 antibody preparations described herein or the anti-PCSK9 antibody disclosed herein. The present invention also relates to use of any of the anti-PCSK9 antibody preparations described herein or the anti-PCSK9 antibody disclosed herein in preparing a drug for inhibiting the binding of PCSK9 to LDL-receptor (LDLR) in a subject.

In another aspect, the present invention relates to a method for lowering the cholesterol level of a subject, wherein the method comprises administering to the subject an effective amount of any of the anti-PCSK9 antibody preparations described herein or the anti-PCSK9 antibody disclosed herein. In one embodiment, cholesterol is LDL-cholesterol, preferably serum cholesterol. In another aspect, the present invention relates to a method for lowering the LDL-cholesterol level of a subject, comprising administering to the subject an effective amount of any of the anti-PCSK9 antibody preparations described herein or the anti-PCSK9 antibody disclosed herein. In some embodiments, the present invention relates to a method for lowering the LDL-cholesterol level in the serum of a subject, wherein the method comprises administering to the subject an effective amount of any of the anti-PCSK9 antibody preparations described herein or the anti-PCSK9 antibody disclosed herein. In another aspect, the present invention also relates to use of any of the anti-PCSK9 antibody preparations described herein or the anti-PCSK9 antibody disclosed herein in preparing a drug for lowering the cholesterol level (LDL-cholesterol level or serum LDL-cholesterol level in one embodiment) in a subject.

In another aspect, the present invention relates to a method for preventing or treating a disorder associated with increased LDL-cholesterol level in a subject, wherein the method comprises administering to the subject an effective amount of any of the anti-PCSK9 antibody preparations described herein or the anti-PCSK9 antibody disclosed herein. The present invention also relates to use of any of the anti-PCSK9 antibody preparations described herein or the anti-PCSK9 antibody disclosed herein in preparing a drug for treating a disorder associated with increased LDL-cholesterol level in a subject.

In one aspect, the present invention relates to a method for preventing or treating a cholesterol-related disease, wherein the method comprises administering to the subject an effective amount of any of the anti-PCSK9 antibody preparations described herein or the anti-PCSK9 antibody disclosed herein. The present invention also relates to use of any of the anti-PCSK9 antibody preparations described herein or the anti-PCSK9 antibody disclosed herein in preparing a drug for treating a cholesterol-related disease. Exemplary and non-limiting examples of the cholesterol-related disease will be provided hereinafter. In some embodiments, the cholesterol-related disease is hypercholesterolemia or hyperlipidaemia. In some embodiments, the present invention relates to a method for treating hypercholesterolemia and/or hyperlipidaemia, wherein the method comprises administering to the subject an effective amount of any of the anti-PCSK9 antibody preparations described herein or the anti-PCSK9 antibody disclosed herein. In some embodiments, the present invention also relates to use of any of the anti-PCSK9 antibody preparations described herein or the anti-PCSK9 antibody disclosed herein in preparing a drug for treating hypercholesterolemia and/or hyperlipidaemia.

In one aspect, the present invention relates to a method for preventing or treating any disease or disorder capable of being improved, alleviated, inhibited or prevented by eliminating, inhibiting, or reducing the activity of PCSK9. In some embodiments, diseases or disorders that can be treated or prevented by using statins can also be treated or prevented by using any of the anti-PCSK9 antibody preparations described herein or the anti-PCSK9 antibody disclosed herein. In some embodiments, diseases or disorders that can benefit from the prevention of cholesterol synthesis or the increase of LDLR expression can also be treated by using any of the anti-PCSK9 antibody preparations described herein or the anti-PCSK9 antibody disclosed herein.

In some embodiments, the method described herein also comprises administering to the subject an effective amount of an additional therapeutic agent in a combination therapy. In one embodiment, the present invention also relates to a combination product that comprises the antibody preparation or the antibody described herein and the additional therapeutic agent. In one embodiment, the additional therapeutic agent can increase the level of LDLR protein. In another embodiment, the additional therapeutic agent can decrease the level of LDL-cholesterol. In another embodiment, the additional therapeutic agent comprises statins. In another embodiment, the additional therapeutic agent is a statin. In some embodiments, the statin is selected from atorvastatin, fluvastatin, lovastatin, mevastatin, pitavastatin, pravastatin, rosuvastatin, simvastatin, and any combination thereof. In certain embodiments, the additional therapeutic agent is used to prevent and/or treat atherosclerosis and/or cardiovascular diseases. In certain embodiments, the additional therapeutic agent is used to lower the risk of cardiovascular event recurrence. In certain embodiments, the additional therapeutic agent is used to increase the level of HDL-cholesterol in a subject. In some embodiments, the subject or individual is a mammal, preferably a human.

The aforementioned combination therapy includes combined administration (wherein two or more therapeutic agents are contained in one preparation or separate preparations) and separate administration, wherein any of the anti-PCSK9 antibody preparations or the anti-PCSK9 antibody disclosed herein can be administered before, concurrently with, and/or after the administration of an additional therapeutic agent and/or an adjuvant.

In order to prevent or treat diseases, the appropriate dosage of the preparation or antibody disclosed herein (used alone or in combination with one or more other additional therapeutic agents) will depend on the type of the disease to be treated, the type of the antibody, the severity and progression of the disease, the purpose for which the antibody is administered (prophylactic or therapeutic), previous treatments, the clinical history of the patient, responses to the preparation or antibody, and the discretion of the attending physician. The antibody is suitably administered to a patient through a single treatment or through a series of treatments. The exemplary dosage range of the antibody includes 3 mg/kg to 30 mg/kg.

In other aspects, the present invention provides use of any of the anti-PCSK9 antibody preparations or the anti-PCSK9 antibody disclosed herein in the production or preparation of a drug used to treat the aforementioned related diseases or disorders.

In certain embodiments, any anti-PCSK9 antibody preparation disclosed herein or the anti-PCSK9 antibody disclosed herein can be prophylactically administered to prevent or alleviate the onset of hypercholesterolemia, hyperlipidemia, cardiovascular diseases, and/or any cholesterol-related disease. In certain embodiments, any anti-PCSK9 antibody preparation disclosed herein or the anti-PCSK9 antibody disclosed herein can be administered to treat existing hypercholesterolemia and/or hyperlipidemia and/or cardiovascular diseases and/or any cholesterol-related disease. In some embodiments, any anti-PCSK9 antibody preparation disclosed herein or the anti-PCSK9 antibody disclosed herein will delay the onset of a disorder and/or symptoms associated with the disorder.

### IV. Others

In one aspect, the present invention provides a nucleic acid encoding any aforementioned anti-PCSK9 antibody or a fragment thereof. In one embodiment, a vector comprising the nucleic acid is provided. In one embodiment, the vector is an expression vector. In one embodiment, a host cell comprising the vector is provided. In one embodiment, the host cell is eukaryotic. In another embodiment, the host cell is selected from a yeast cell, a mammalian cell, or other cells suitable for preparing an antibody or an antigen-binding fragment thereof. In another embodiment, the host cell is prokaryotic.

In one embodiment, the present invention provides a method for preparing an anti-PCSK9 antibody or a fragment thereof (preferably an antigen binding fragment), wherein the method comprises culturing the host cell under conditions suitable for the expression of the nucleic acid encoding the antibody or the fragment thereof (preferably the antigen-binding fragment), and optionally isolating the antibody or the fragment thereof (preferably the antigen-binding fragment). In a certain embodiment, the method further comprises isolating the anti-PCSK9 antibody or the fragment thereof (preferably the antigen-binding fragment) from the host cell.

In one aspect, the present invention relates to a method for detecting PCSK9 protein in a sample, wherein the method comprises (a) contacting the sample with any of the anti-PCSK9 antibody preparations described herein or the antibody disclosed herein; and (b) detecting the formation of a complex between the anti-PCSK9 antibody or fragment thereof and the PCSK9 protein. In one embodiment, the anti-PCSK9 antibody is detectably labeled.

The present invention also encompasses any combination of the embodiments described herein. Any of the embodiments described herein or any combination thereof is applicable to any and all of the preparations or anti-PCSK9 antibodies or fragments thereof, the methods and the use described herein.

### BRIEF DESCRIPTON OF THE DRAWINGS:

FIG. 1 shows the capability of anti-PCSK9 antibodies at different concentrations in blocking the binding of PCSK9 to LDLR.
FIG. 2 shows the capability of anti-PCSK9 antibodies at different concentrations in enhancing the restoration of LDLR in HepG2 cells.
FIG. 3 shows the capability of anti-PCSK9 antibodies at different concentrations in reducing the internalization of LDLR into cells.

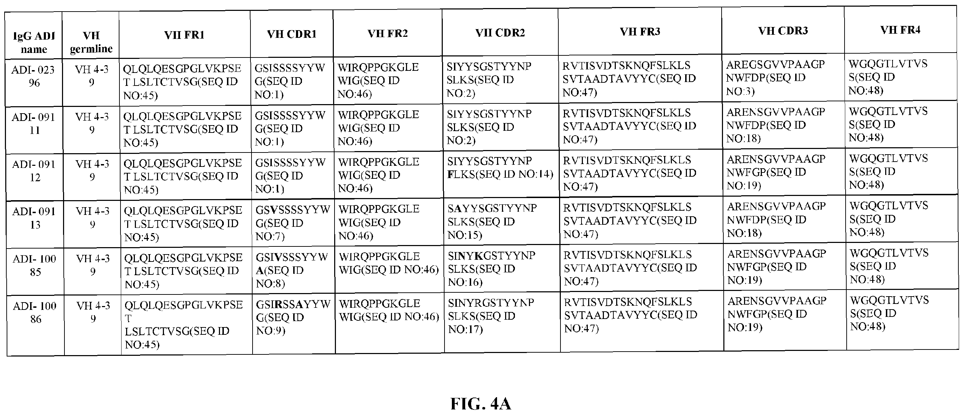

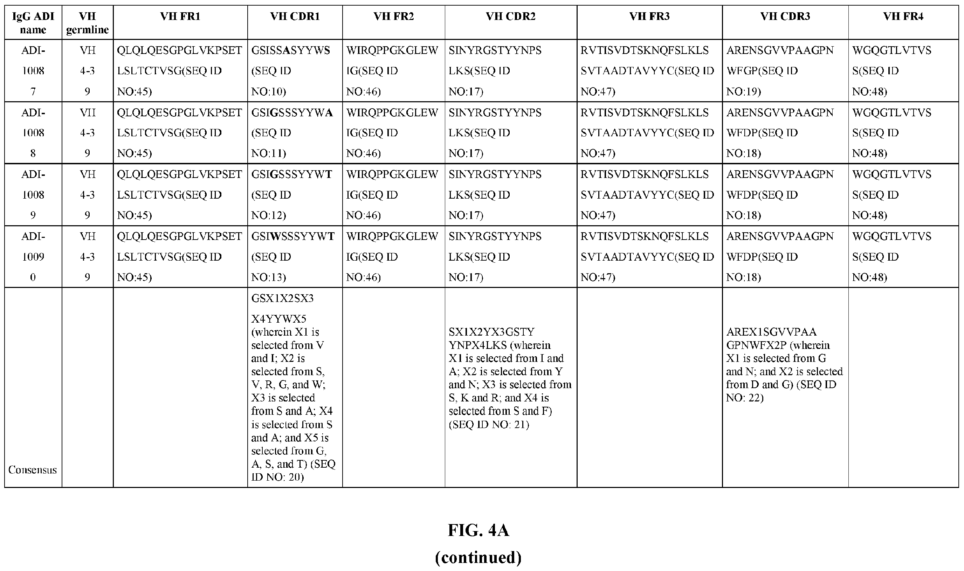

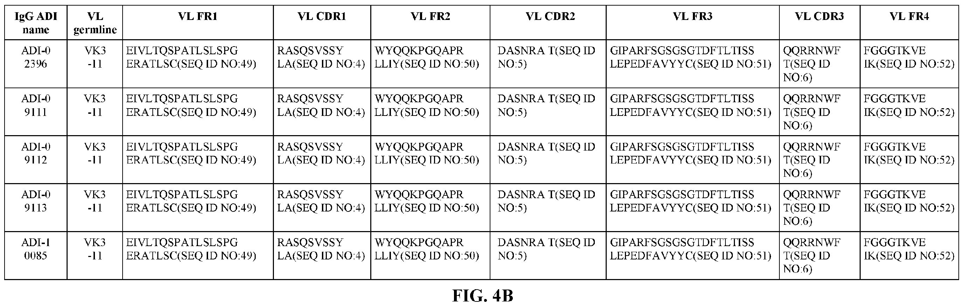

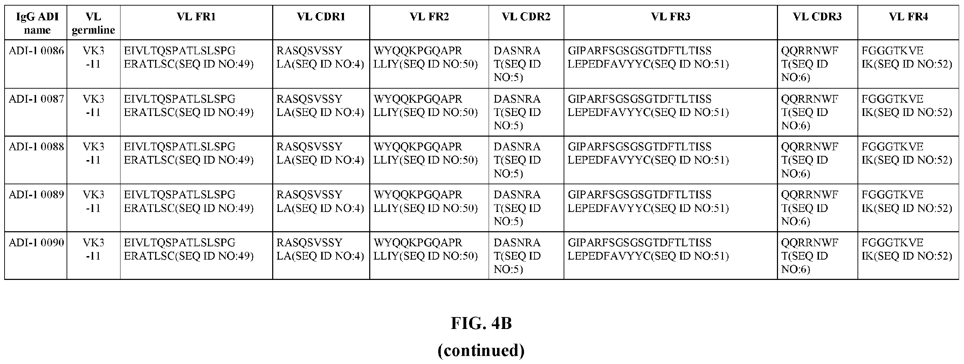

FIG. 4A shows the sequence information of FRs and CDRs of heavy chains of exemplary antibodies of the present invention, and FIG. 4B shows the sequence information of FRs and CDRs of light chains of exemplary antibodies of the present invention.

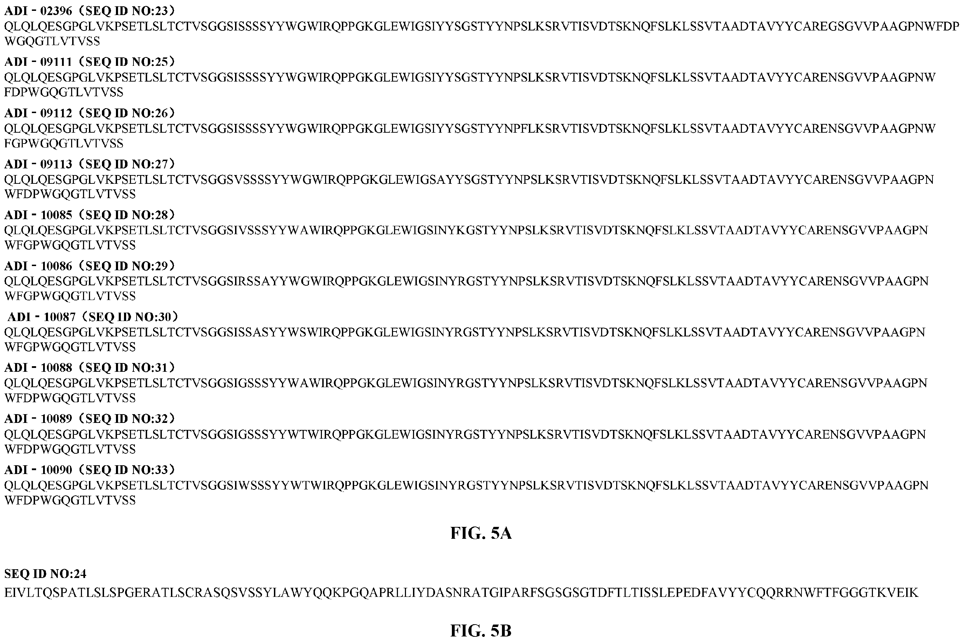

FIG. 5A shows the sequence information of heavy chain variable regions of exemplary antibodies of the present invention, and FIG. 5B shows the sequence information of a light chain variable region of an exemplary antibody of the present invention.
FIG. 6 shows a graph of the % change rate (average) of LDL-C in serum relative to a pre-administration level (before D1 administration, baseline) after subcutaneous or intravenous administration of an anti-PCSK9 antibody or Evolocumab to rats plotted against time.
FIG. 7 shows a graph of the % change rate (average) of % HDL-C in serum relative to a pre-administration level (before D1 administration, baseline) after subcutaneous or intravenous administration of the anti-PCSK9 antibody or Evolocumab to rats plotted against time.
FIG. 8 shows a graph of the % change rate (average) of LDL-C in serum relative to a pre-administration level (before D1 administration, baseline) after administration of the anti-PCSK9 antibody or Evolocumab to cynomolgus monkeys plotted against time.
FIG. 9 shows a graph of the % change rate (average) of HDL-C in serum relative to a pre-administration level (before D1 administration, baseline) after administration of the anti-PCSK9 antibody or Evolocumab to cynomolgus monkeys plotted against time.
FIG. 10 shows a graph of the % change rate (average) of serum TC relative to a pre-administration level (before D1 administration, baseline) after administration of the anti-PCSK9 antibody or Evolocumab to cynomolgus monkeys plotted against time.

### Definitions

The terms used in the present invention have the following definitions. If no definitions are given herein, the terms used in the present invention have the meanings commonly understood in the art.

For the purpose of explaining this specification, the following definitions will be used, and wherever appropriate, terms used in the singular form may also include the plural form, and *vice versa.* It should be understood that the terminology used herein is for the purpose of describing specific embodiments only, and is not intended to be limiting.

As used herein, the term "and/or" refers to any one of the options or two or more of the options.

As used herein, the term "comprise" or "include" is intended to mean that the described elements, integers or steps are included, but not to the exclusion of any other elements, integers or steps. The term "comprise" or "include" used herein, unless otherwise specified, also encompasses the situation where the entirety consists of the described elements, integers or steps. For example, when referring to "comprise" an antibody variable region of a particular sequence, it is also intended to encompass an antibody variable region consisting of the specific sequence.

Unless otherwise stated, the term "proprotein convertase subtilisin/kexin type 9 (PCSK9)", "PCSK9", or "NARC-1" used herein refers to any natural PCSK9, preferably any natural PCSK9 from any vertebrate (including mammals, such as primates (e.g., humans) and rodents (e.g., mice and rats)). This term encompasses "full-length" unprocessed PCSK9 and PCSK9 in any form resulting from intracellular processing or any fragment thereof. This term also includes variants of naturally existing PCSK9, such as splice variants or allelic variants.

The term "activity of PCSK9" or "bioactivity of PCSK9" used herein includes any biological effect of PCSK9. In some embodiments, the activity of PCSK9 includes the ability of PCSK9 to interact with or bind to a substrate or a receptor. In some embodiments, the bioactivity of PCSK9 is the ability of PCSK9 to bind to LDL-receptor (LDLR). In some embodiments, PCSK9 binds to LDLR and catalyzes a reaction involving LDLR. In some embodiments, the activity of PCSK9 includes the ability of PCSK9 to decrease or reduce the availability of LDLR. In some embodiments, the bioactivity of PCSK9 includes the ability of PCSK9 to increase the amount of LDL in a subject. In some embodiments, the bioactivity of PCSK9 includes the ability of PCSK9 to reduce the amount of LDLR capable of binding to LDL in a subject. In some embodiments, the bioactivity of PCSK9 includes the ability of PCSK9 to reduce the amount of LDLR capable of binding to LDL. In some embodiments, the bioactivity of PCSK9 includes any bioactivity caused by PCSK9 signaling.

The term "anti-PCSK9 antibody", "anti-PCSK9", "PCSK9 antibody" or "antibody binding to PCSK9" refers to an antibody capable of binding to a PCSK9 protein or a fragment thereof with sufficient affinity so as to serve as a diagnostic agent and/or a therapeutic agent in targeting PCSK9. In one embodiment, an anti-PCSK9 antibody binds to an unrelated, non-PCSK9 protein to an extent less than about 10% of the extent to which the antibody binds to PCSK9, as measured, for example, by radioimmunoassay (RIA). In some embodiments, the equilibrium dissociation constant (K_{D}) of an anti-PCSK9 antibody is less than or equal to 1 µM, 100 nM, 10 nM, 1 nM, 0.1 nM, 0.01 nM, or 0.001 nM (*e.g.,* 10⁻⁸ M or less, *e.g.,* 10⁻⁸ M to 10⁻¹³ M, *e.g.,* 10⁻⁹ M to 10⁻¹³ M).

"Antibody fragment" refers to a molecule different from an intact antibody, which comprises a portion of the intact antibody and binds to an antigen to which the intact antibody binds. Examples of the antibody fragment include, but are not limited to, Fv, Fab, Fab', Fab'-SH, F(ab')2; a diabody; a linear antibody; a single-chain variable fragment (*e.g.,* scFv); a single-domain antibody; a bivalent or bispecific antibody or a fragment thereof; a Camelidae antibody; and a bispecific antibody or multispecific antibody formed from antibody fragments.

As used herein, the term "epitope" refers to a moiety of an antigen (*e.g.,* PCSK9) that specifically interacts with an antibody molecule.

An "antibody that binds to the same or overlapping epitope" as a reference antibody refers to an antibody that blocks 50% or more of the binding of the reference antibody to its antigen in a competition assay. Conversely, the reference antibody blocks 50% or more of the binding of the antibody to its antigen in a competition assay.

An antibody that competes with a reference antibody to bind to its antigen refers to an antibody that blocks more than 50%, 60%, 70%, 80%, 90%, or 95% of the binding of the reference antibody to its antigen in a competition assay. Conversely, the reference antibody blocks more than 50%, 60%, 70%, 80%, 90%, or 95% of the binding of the antibody to its antigen in a competition assay. Numerous types of competitive binding assays can be used to determine whether an antibody competes with another, such as direct or indirect solid-phase radioimmunoassay (RIA), direct or indirect solid-phase enzyme immunoassay (EIA), and sandwich competition assay (see, e.g., Stahli et al., 1983, Methods in Enzymology 9: 242-253).

An antibody that inhibits (*e.g.,* competitively inhibits) the binding of a reference antibody to its antigen refers to an antibody that inhibits more than 50%, 60%, 70%, 80%, 90%, or 95% of the binding of the reference antibody to its antigen. Conversely, the reference antibody inhibits more than 50%, 60%, 70%, 80%, 90%, or 95% of the binding of the antibody to its antigen. The binding of an antibody to its antigen can be measured by affinity (*e.g.,* equilibrium dissociation constant). Methods for determining affinity are known in the art.

An antibody that shows the same or similar binding affinity and/or specificity as a reference antibody refers to an antibody that is capable of having at least more than 50%, 60%, 70%, 80%, 90%, or 95% of the binding affinity and/or specificity of the reference antibody. This can be determined by any method known in the art for determining binding affinity and/or specificity.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact site"). CDRs are primarily responsible for binding to antigen epitopes. The CDRs of heavy and light chains are generally referred to as CDR1, CDR2, and CDR3, which are numbered sequentially from N-terminus. The CDRs located in a heavy chain variable domain of an antibody are referred to as HCDR1, HCDR2, and HCDR3, whereas the CDRs located in a light chain variable domain of an antibody are referred to as LCDR1, LCDR2, and LCDR3. In a given amino acid sequence of a light chain variable region or a heavy chain variable region, the exact amino acid sequence boundary of each CDR can be determined using any one or a combination of many well-known antibody CDR assignment systems including, *e.g.,* Chothia based on the three-dimensional structure of antibodies and the topology of the CDR loops (Chothia et al. (1989) Nature 342:877-883; Al-Lazikani et al., Standard conformations for the canonical structures of immunoglobulins, Journal of Molecular Biology, 273:927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

However, it should be noted that boundaries of CDRs of variable regions of an antibody based on different assignment systems may differ. That is, CDR sequences of variable regions of an antibody defined by different assignment systems differ. Therefore, when it comes to defining an antibody with specific CDR sequences defined in the present invention, the scope of antibody also encompasses such antibodies whose variable region sequences comprise the specific CDR sequences, but having claimed CDR boundaries different from the specific CDR boundaries defined by the present invention due to a different protocol (*e.g.,* different assignment system rules or their combinations) applied.

Antibodies with different specificities (*i.e.,* different binding sites for different antigens) have different CDRs. However, although CDRs differ from antibody to antibody, only a limited number of amino acid positions within the CDRs are directly involved in antigen binding. The smallest overlapping region can be determined using at least two of the Kabat, Chothia, AbM, Contact, and North methods, thereby providing a "minimal binding unit" for antigen binding. The minimal binding unit may be a sub-portion of the CDR. As will be clear to those skilled in the art, residues of the rest CDR sequences can be determined via antibody structure and protein folding. Therefore, any variants of the CDRs given herein are also considered in the present invention. For example, in a CDR variant, the amino acid residues in the minimal binding unit may remain unchanged, while the other CDR residues defined by Kabat or Chothia may be substituted by conservative amino acid residues.

"Functional Fc region" possesses the "effector functions" of Fc regions of native sequences. Exemplary "effector functions" include C1q binding, CDC, Fc receptor binding, ADCC, phagocytosis, cell surface receptor (*e.g.,* B cell receptor, or BCR) down-regulation, and the like. Such effector functions generally require that the Fc region is associated with a binding domain (*e.g.,* an antibody variable domain) and can be assessed using a variety of assays, such as those disclosed herein.

The term "Fc region" is used herein to define a C-terminus region of an immunoglobulin heavy chain, which comprises at least one portion of a constant region. The term includes Fc regions of native sequences and variant Fc regions. In certain embodiments, a human IgG heavy chain Fc region extends from Cys226 or Pro230 to the carbonyl end of the heavy chain. However, the C-terminal lysine (Lys447) of the Fc region may or may not be present. Unless otherwise stated, the numbering of amino acid residues in the Fc region or constant region is based on an EU numbering system, which is also called EU index as described in Kabat et al., Sequences of Proteins of Immunological Interest, 5th Ed. Public Health Service, National Institutes of Health, Bethesda, MD, 1991.

"Antibodies and antigen-binding fragments thereof" applicable to the present invention include but are not limited to polyclonal, monoclonal, monovalent, bispecific, isoconjugate, multispecific, recombinant, heterogenous, heterogenous hybrid, chimeric, humanized (particularly CDR-grafted), deimmunized or human antibodies, Fab fragments, Fab' fragments, F(ab')₂ fragments, fragments produced by an Fab expression library, Fd, Fv, disulphide-linked Fv (dsFv), single-chain antibodies (such as scFv), diabodies or tetrabodies (Holliger P. et al. (1993) Proc.Natl.Acad.Sci.U.S.A.90(14), 6444-6448), nanobodies (also referred to as single-domain antibodies), anti-idiotype (anti-Id) antibodies (including, for example, anti-Id antibodies against the antibody disclosed herein), and an epitope-binding fragment of any of the above.

"Human consensus framework" refers to a framework which represents the most common amino acid residues in the selection of human immunoglobulin VL or VH framework sequences. Generally, the selection of human immunoglobulin VL or VH sequences is a selection from subtypes of variable domain sequences.

"Antibody in IgG form" refers to the heavy chain constant region of the antibody belonging to the IgG form. Heavy chain constant regions of all antibodies of the same type are identical, and heavy chain constant regions of antibodies of different types are different. For example, an antibody in the form of IgG1 refers to the Ig domain of its heavy chain constant region being an Ig domain of an IgG1.

The term "therapeutic agent" described herein encompasses any substance that is effective in preventing or treating cholesterol-related diseases, including cytotoxic agents, other antibodies, small molecule drugs, or immunomodulatory agents, *etc.*

"Human antibody" refers to an antibody having an amino acid sequence which corresponds to the amino acid sequence of an antibody generated by a human or human cell or derived from a non-human source that utilizes human antibody libraries or other human antibody encoding sequences. This definition of a human antibody explicitly excludes humanized antibodies comprising non-human antigen-binding residues.

"Humanized" antibody refers to a chimeric antibody comprising amino acid residues from non-human CDRs and amino acid residues from human FRs. In some embodiments, a humanized antibody will comprise substantially all of at least one, typically two variable domains, wherein all or substantially all CDRs correspond to those of a non-human antibody, and all or substantially all FRs correspond to those of a human antibody. A humanized antibody may optionally comprise at least a portion of an antibody constant region derived from a human antibody. The "humanized form" of an antibody (such as a non-human antibody) refers to an antibody that has been humanized.

"Individual" or "subject" includes mammals. The mammals include, but are not limited to, domestic animals (*e.g.,* cattle, goats, cats, dogs, and horses), primates (*e.g.,* human and non-human primates such as monkeys), rabbits, and rodents (*e.g.,* mice and rats). In some embodiments, the individual or subject is a human.

An "isolated" antibody is an antibody which has been separated from components of its natural environment. In some embodiments, the antibody is purified to a purity greater than 95% or 99% as determined by, *e.g.,* electrophoresis (*e.g.,* SDS-PAGE, isoelectric focusing (IEF) and capillary electrophoresis) or chromatography (*e.g.,* ion exchange or reverse-phase HPLC). For a review of methods for assessing antibody purity, see, for example, Flatman et al., J. Chromatogr., B848:79-87 (2007).

The "percent (%) amino acid sequence identity" relative to a reference polypeptide sequence is defined as the percentage of amino acid residues in a candidate sequence that are identical to those in a reference polypeptide sequence after aligning the sequences (with gaps introduced if necessary) to achieve maximum percent sequence identity without considering any conservative replacement as part of sequence identity. Various methods in the art can be employed to perform sequence alignment so as to determine the percent amino acid sequence identity, for example, using computer software available to the public, such as BLAST, BLAST-2, ALIGN, or MEGALIGN (DNASTAR) software. Those skilled in the art can determine suitable parameters for measuring alignment, including any algorithm required to obtain maximum alignment for the full length of the aligned sequences.

Additionally or alternatively, the nucleic acid sequences and protein sequences described herein can be further used as "query sequences" to perform searches against public databases to, e.g., identify other family member sequences or related sequences.

The term "combination product" refers to a kit with a fixed combination, a non-fixed combination, or a part for combined administration in the form of a dose unit, wherein two or more therapeutic agents can be independently administered simultaneously or separately administered at intervals of time, especially when these intervals allow combination partners to exhibit collaboration, such as synergistic effect. The term "fixed combination" means that the antibody disclosed herein and a combination partner (*e.g.,* other therapeutic agents, such as statin drugs) are simultaneously administered to a patient in the form of a single entity or dose. The term "non-fixed combination" means that the antibody disclosed herein and a combination partner (*e.g.,* other therapeutic agents, such as statins) are simultaneously, concurrently, or sequentially administered to a patient as separate entities, without specific time limitation, wherein such administration provides therapeutically effective levels of the two compounds in the patient. The latter is also applicable to a cocktail therapy, *e.g.,* administration of three or more therapeutic agents. In one preferred embodiment, the drug combination is a non-fixed combination.

The term "combination therapy" means that two or more therapeutic agents are administered to treat cholesterol-related diseases as described herein. Such administration includes co-administration of these therapeutic agents in a substantially simultaneous manner, for example, in a single capsule with a fixed proportion of active ingredients. Alternatively, such administration includes co-administration of each active ingredient in a variety of or separate containers (such as tablets, capsules, powder and liquid). The powder and/or liquid can be reconstituted or diluted to a desired dosage before administration. In addition, such administration also includes using each type of therapeutic agents in a sequential manner at approximately the same time or at different times. In any case, the therapeutic regimen will provide the beneficial effect of the drug combination in the treatment of disorders or symptoms described herein.

As used herein, "treatment" (or "treat" or "treating") refers to slowing, interrupting, arresting, alleviating, stopping, reducing, or reversing the progression or severity of an existing symptom, disorder, condition, or disease.

As used herein, "prevention" (or "prevent" or "preventing") includes the inhibition of the onset or progression of a disease or disorder or a symptom of a specific disease or disorder. In some embodiments, subjects with family histories are candidates for prophylactic regimens. Generally, the term "prevention" (or "prevent" or "preventing") refers to the administration of a drug prior to the onset of disorders or symptoms, particularly in subjects at risk.

The term "cholesterol-related disease" includes any one or more of the following: hypercholesterolemia, hyperlipidaemia, heart disease, metabolic syndrome, diabetes mellitus, coronary heart disease, stroke, cardiovascular diseases, Alzheimers disease, and general dyslipidemia (shown as, for example, increased total serum cholesterol, increased LDL, increased triglyceride, increased VLDL, and/or low HDL). Some non-limiting examples of primary and secondary dyslipidemias that can be treated with an anti-PCSK9 antibody (alone or in combination with one or more other drugs) include metabolic syndrome, diabetes mellitus, familial combined hyperlipidemia, familial hypertriglyceridemia, familial hypercholesterolemias, including heterozygous hypercholesterolemia, homozygous hypercholesterolemia, and familial defective apolipoprotein B-100; polygenic hypercholesterolemia; remnant removal disease; hepatic lipase deficiency; dyslipidemia secondary to any of the following: dietary indiscretion, hypothyroidism, drugs (including estrogen and progesterone therapies, β blockers and thiazide diuretics); nephrotic syndrome, chronic renal failure, Cushing's syndrome, primary biliary cirrhosis, glycogen storage diseases, hepatoma, cholestasis, acromegaly, insulinoma, isolated growth hormone deficiency, and alcohol-induced hypertriglyceridemia.

The term "hypercholesterolemia" used herein refers to a disorder in which the cholesterol level rises to above a certain level. In some embodiments, the LDL-cholesterol level rises to above a certain level. In some embodiments, the serum LDL-cholesterol level rises to above a certain level.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operably linked. Such vectors are called "expression vectors" herein.

"Subject/patient sample" refers to a collection of cells or fluids obtained from a patient or a subject.

The source of tissue or cell samples can be solid tissues, *e.g.,* from fresh, frozen and/or preserved organ or tissue samples or biopsy samples or puncture samples; blood or any blood component; body fluids such as cerebrospinal fluids, amniotic fluids, peritoneal fluids, or interstitial fluids; and cells from a subject at any time during pregnancy or development. Tissue samples may comprise compounds which are naturally not mixed with tissues, such as preservatives, anticoagulants, buffers, fixatives, nutrients, antibiotics, and the like.

The term "effective amount" refers to an amount or a dosage of the preparation, antibody, or fragment described herein which generates an expected effect in a treated patient after the administration of a single or multiple doses. The effective amount can be easily determined by an attending physician as a person skilled in the art by considering a variety of factors as follows: species such as mammals; its size, age, and general health condition; the specific disease involved; the extent or severity of the disease; response in an individual patient; specific antibody administered; route of administration; bioavailability characteristics of the administered preparation; selected dosage regimen; and use of any concomitant therapy.

The "therapeutically effective amount" refers to an amount effective to achieve a desired therapeutic result at a necessary dosage for a necessary period of time. The therapeutically effective amount of the preparation, antibody or antibody fragment described herein, or conjugate or composition thereof can vary depending on a variety of factors such as disease state, age, sex and weight of an individual, and the ability of the antibody or antibody portion to elicit a desired response in the individual. The therapeutically effective amount is also such an amount in which any toxic or undesired effect of the preparation, antibody or antibody fragment or conjugate or composition thereof is inferior to the therapeutically beneficial effect.

The "prophylactically effective amount" refers to an amount effective to achieve a desired prophylactic result at a necessary dosage for a necessary period of time. Generally, since a prophylactic dose is administered in a subject before or at an earlier stage of a disease, a prophylactically effective amount will be less than a therapeutically effective amount.

The terms "host cell", "host cell line" and "host cell culture" are used interchangeably and refer to cells into which exogenous nucleic acids are introduced, including progenies of such cells. Host cells include "transformants" and "transformed cells", which include primary transformed cells and progenies derived therefrom, regardless of the number of passages. A generation may not be completely identical in nucleic acid content to the parent cell, but may contain mutations. Mutant generations having the same function or bioactivity that are screened or selected from the initially transformed cells are included herein.

As used herein, the term "preparation" refers to a composition which is suitably administered to animals, preferably mammals (including humans) and comprises at least one active ingredient and at least one non-active ingredient. "Liquid preparation" refers to a preparation in the form of liquid. The liquid preparation disclosed herein comprises (i) an anti-PCSK9 antibody or a fragment thereof; (ii) a buffer; (iii) a viscosity inhibitor; (iv) a surfactant; and (v) a solvent. The composition of the preparation disclosed herein may be as shown in the aforementioned liquid preparation embodiments. The liquid preparation disclosed herein is preferably an injection, more preferably a subcutaneous injection.

As used herein, the "buffer" refers to a pH buffer. Preferably, the buffer can keep pH of the liquid preparation disclosed herein at about 5.0 to 6.0, preferably about 5.2 to 5.8, more preferably about 5.4 to 5.6, and most preferably about 5.5. The concentration of the buffer in the liquid preparation is, for example, about 0.01 mg/mL to 50 mg/mL, about 0.1 mg/mL to 50 mg/mL, about 0.2 mg/mL to 10 mg/mL, about 0.5 mg/mL to 2.5 mg/mL, about 1.0 mg/mL to 2.0 mg/mL, or about 1.5 mg/mL. Preferably, the buffer is selected from histidine, glutamate, phosphate, acetate, citrate and tris(hydroxymethyl)aminomethane.

As used herein, the "viscosity inhibitor" refers to a substance that can reduce the viscosity of the liquid preparation comprising an anti-PCSK9 antibody. The concentration of the viscosity inhibitor in the liquid preparation is, for example, about 10 mmol/L to 1000 mmol/L, about 20 mmol/L to 500 mmol/L, about 50 mmol/L to 300 mmol/L, or about 50 mmol/L to 200 mmol/L. Preferably, the viscosity inhibitor is selected from sugar alcohol, arginine, arginine hydrochloride, sodium thiocyanate, ammonium thiocyanate, ammonium sulfate, ammonium chloride, calcium chloride, zinc chloride, sodium acetate, and combinations thereof. More preferably, the viscosity inhibitor is sorbitol or a combination of sorbitol and arginine or arginine salt (preferably arginine hydrochloride). Preferably, the concentration of sorbitol in the liquid preparation is about 1% to 6% (w/v); and the concentration of arginine or arginine salt (preferably arginine hydrochloride) in the liquid preparation is about 50 mmol/L to 180 mmol/L, preferably about 70 mmol/L to 150 mmol/L, and more preferably about 90 mmol/L.

As described herein, the "surfactant" refers to a substance which can significantly change the interface state of a solution system after being added in a small amount. The concentration of the surfactant in the liquid preparation is, for example, about 0.01 mg/mL to 5 mg/mL, about 0.05 mg/mL to 1 mg/mL, or about 0.1 mg/mL to 0.5 mg/mL, and more preferably about 0.3 mg/mL. Preferably, the surfactant is a nonionic surfactant, such as pluronics, polysorbate-80, polysorbate-60, polysorbate-40, or polysorbate-20.

As used herein, the term "solvent" refers to a liquid that is used to dissolve or suspend active ingredients and non-active ingredients to form a liquid preparation. Solvents that can be used in the present invention include, but are not limited to, water for injection, organic solvents for injection (including but not limited to oil for injection, ethanol, and propylene glycol), and combinations thereof.

As used herein, the term "sugar alcohol" refers to a corresponding polyol which is obtained by reduction reaction, such as catalytic hydrogenation, of monosaccharide. Sugar alcohols include, but are not limited to, sorbitol, mannitol, erythritol, maltitol, lactitol, xylitol, *etc.*

The term "about" used in combination with a numerical value is intended to encompass the numerical values in a range from a lower limit less than the specified numerical value by 10% to an upper limit greater than the specified numerical value by 10%.

As used herein, "w/v" refers to "weight/volume", for example, "1% w/v" means 1 g/100 mL = 0.01 g/mL = 10 mg/mL.

### DETAILED DESCRIPTION

The present invention will be further illustrated below with reference to examples, and the following examples should not be construed as limiting the present invention. The scope of the present invention is defined by the claims.

### Abbreviations:

SEC-HPLC: size exclusion chromatography-high performance liquid chromatography
CE-SDS: capillary electrophoresis-sodium dodecyl sulfate
CZE: capillary zone electrophoresis

### Example 1. Determination of Parent Antibody by Screening of Anti-PCSK9 Antibodies

### Antigen Biotin Label

According to the instructions of the manufacturer, PCSK9 antigen (SEQ ID NO: 53) was labeled with biotin using the sulfosuccinimidylbiotin labeling kit from Pierce. The FITC-labeled goat anti-human immunoglobulin F(ab') kappa chain antibody (LC-FITC) was purchased from Southern Biotech, polyethylene avidin (SA-PE) was purchased from Sigma, and streptavidin-633 (SA-633) was purchased from Molecular Probes. Streptomycin microbeads and a cellular immunomagnetic bead separation column were purchased from Miltenyi LS.

### Preliminary Screening

Eight synthetic yeast-based antibody presentation libraries (from Adimab) were amplified according to an existing method (Xu, et al., 2013; WO 2009036379; WO 2010105256; WO 2012009568), with the diversity of each library reaching 1 × 10⁹. Briefly, the first two rounds of screening employed magnetic-activated cell sorting using the MACS system available from Miltenyi. First, yeast cells (approximately 1×10¹⁰ cells/library) from each library were incubated in FACS buffer (phosphate buffer, containing 0.1% bovine serum albumin) for 15 min at room temperature, and the buffer contained 100 nM biotin-labeled PCSK9 antigen as prepared above. The cells were washed once with 50 mL of pre-cooled FACS buffer and resuspended in 40 mL of the same buffer, followed by addition of 500 µL of streptomycin microbeads and incubation at 4 °C for 15 min. The mixture was centrifuged at 1000 rpm for 5 min. After discarding the supernatant, the cells were resuspended in 5 mL of FACS buffer. The resulting cell suspension was loaded on a Miltenyi LS column. After loading, the column was washed three times with FACS buffer, 3 mL each time. The Miltenyi LS column was removed from the magnetic field and eluted with 5 mL of growth medium. The eluted yeast cells were collected and incubated overnight at 37 °C.

The next round of sorting was performed using a flow cytometer, wherein approximately 1 × 10⁸ yeast cells screened by the MACS system were washed three times with FACS buffer and cultured in PCSK9 antigens or PCSK9-Fc fusion antigens labeled by a low concentration of biotin (100-1 nM) at room temperature. The culture media were discarded. After the cells were washed twice with FACS buffer, the cells were mixed with LC-FITC (1:100 dilution) and SA-633 (1:500 dilution) or EA-PE (1:50 dilution) and cultured at 4 °C for 15 min. The cells were washed twice with pre-cooled FACS buffer, resuspended in 0.4 mL of buffer and transferred into a separator tube with a filter. The cells were sorted using FACS ARIA (BD Biosciences).

Several rounds of screening were then performed to give competitive ligands and remove nonspecific conjugates (such as the membrane protein of CHO cells). After the last several rounds of sorting, the collected yeast cells were plated and cultured at 37 °C overnight, and target monoclonal antibodies were picked out. Sanger sequencing was employed to sequence variable regions of the obtained antibodies. About 310 antibodies with unique variable region sequences were obtained and identified one by one.

These anti-PCSK9 antibody proteins were obtained by yeast expression and protein A affinity chromatography purification.

### Production and Purification of Antibodies

The yeast cells expressing the anti-PCSK9 antibodies obtained by screening were induced by shaking at 30 °C for 48 h to express the anti-PCSK9 antibodies. After the induction, the yeast cells were centrifuged at 1300 rpm for 10 min, and the supernatant was collected. The anti-PCSK9 antibodies in the supernatant were purified with Protein A and eluted with acetic acid buffer at pH 2.0, and the anti-PCSK9 antibodies were harvested. The antibodies were digested with papain and purified with KappaSelect (GE Healthcare) to produce corresponding Fab fragments.

According to a conventional method in the art, a gene DNA encoding the anti-PCSK9 antibodies was obtained from the aforementioned yeast cells expressing the anti-PCSK9 antibodies and, based on the conventional method, the gene DNA was cloned to a new expression vector (pCDNA3.1). The aforementioned expression vector containing a target antibody gene and a transfection reagent Lipofectamine TM2000 (purchased from Invitrogen) was transiently transfected into cultured human embryonic kidney cells 293 according to a scheme provided by the manufacturer. The medium was discarded, and the cells were diluted to 4 × 10⁶/mL with fresh medium. The cells were cultured at 37 °C, 5% CO₂ for 7 days, with fresh medium fed every 48 h. After 7 days, the cells were centrifuged at 13000 rpm for 20 min. The supernatant was purified with Protein A to achieve an antibody purity of greater than 95%.

### ForteBio KD Assay (Bio-layer Interferometry)

The ForteBio affinity assay was performed according to the prior art (Estep, P, et al., High throughput solution based measurement of antibody-antigen affinity and epitope binning. MAbs, 2013.5(2): p. 270-8). Briefly, a sensor was equilibrated offline in assay buffer for 30 min, and was equilibrated online for 60 s to establish a baseline. The purified antibody obtained as described above was loaded on line onto an AHQ sensor. The sensor was then exposed to 100 nM PCSK9 antigens for 5 min, before transferring the sensor to the assay buffer for dissociation for 5 min. The kinetic analysis was performed using a 1:1 binding model.

### MSD-SETKinetic Assay

The assay of equilibrium affinity is as described above (Estep, *et al.,* 2013). The biotin-labeled PCSK9 antigen (b-PCSK9) with a final concentration of 10-100 pM obtained above was added to phosphate-buffered saline (PBSF) containing 0.1% IgG-free BSA, and the anti-PCSK9 Fab or mAb obtained above was serially diluted 3 to 5 folds to give an Fab or mAb solution with a concentration of 5-100 nM. The antibodies (diluted in 20 nM phosphate-buffered saline) were coated on a MSD-ECL plate at 4 °C overnight or at room temperature for 30 min. 3% BSA was added, and the antibodies were immobilized at 700 rpm for 30 min at room temperature, and were then washed 3 times with a washing buffer (PBSF + 0.05% Tween 20). The samples were added to the plate, incubated at 700 rpm in a shaker at room temperature for 150 s and then washed once. The plate was added with 250 ng/mL sulfotag-labeled streptomycin (diluted in PBSF) and incubated at room temperature for 3 min. After washing the plate 3 times with the buffer, antigens binding on the plate were identified using MSD Sector Imager 2400. The percentage of unbinding antigens was obtained by the antibody titration method, and it was discovered that the binding of the anti-PCSK9 Fab or mAb to the antigens follows a quadratic equation of pharmacokinetics.

### Identification of Epitope Binding by Octet Red384

The identification of epitope binding adopted a standard sandwich interactive blocking analysis method. A target-specific control IgG was fixed on an AHQ sensor, and an irrelevant human IgG1 antibody was used to occupy an empty Fc-binding site on the sensor. The sensor was immersed in 100 nM target antigen PCSK9 solution for 120 s, and was then put into a second 100 nM anti-PCSK9 antibody or ligand solution prepared as above. Data were read and processed by ForteBio data analysis software 7.0 (ForteBio). If the antigen could be bound by a second antibody or ligand after being bound by an antibody, it was suggested that there was an unbound epitope (non-competitive), otherwise epitope blocking (competitive or ligand blocking) was indicated. Through the aforementioned screening and identification, some antibodies that can block the binding of PCSK9 to LDLR and bind to both human and murine PCSK9 were obtained. To obtain an anti-PCSK9 antibody with higher affinity, the antibody ADI-02396 was optimized by the following method.

### Example 2. Optimization of Affinity of Anti-PCSK9 Antibody

### VHmut Screening

This method was to introduce mutations into antibody heavy chain regions by the conventional mismatched PCR method. In the PCR process, the probability of base pair mismatch was increased to about 0.01 bp by adding 1 µM highly mutated base analogs dPTP and 8-oxo-dGTP.

The resulting mismatched PCR product was constructed into a vector containing a heavy chain constant region by homologous recombination. By this method, a secondary pool with the capacity of 1 × 10⁷ was obtained under screening pressure including PCSK9 antigen titer, unlabeled antigen competition, and competition with the parent antibody. Three rounds of screening were successfully performed by FACS.

### CDRH1/CDRH2 Screening

CDRH3 genes of progeny antibodies obtained by VHmut were constructed into a CDRH1/CDRH2 gene pool with the diversity of 1 × 10⁸, and 3 rounds of screening were carried out for the genes. The first round of screening adopted MACS, while the second and third rounds adopted FACS. Antibody-antigen conjugates were subjected to pressurized screening for screening out antibodies with the highest affinity.

The first round of optimization: The first step was to increase the affinity of the anti-PCSK9 antibody ADI-02396 (named "parent" antibody) with human-murine cross-activity and ligand competition. In short, mutations were introduced into the parent antibody (by employing the "mismatched PCR" method) to establish a secondary yeast-based antibody presentation library. Finally, a secondary library with a size of about 1 × 10⁷ was generated for the subsequent enriching of antibodies with higher affinity. Screening pressure included PCSK9 antigen titer, unlabeled antigen competition, and competition with the parent antibody. The FACS technique was also used to screen a target population (see Chao, et al., Nature Protocols, 2006). After 2 to 3 rounds of enriching, the obtained yeasts were plated to give monoclonal antibodies. After this, 3 progenies with improved affinity, ADI-09111, ADI-09112 and ADI-09113, were obtained. Assayed by ForteBio Octet, the K_{D} ranges of the three antibodies were 1 nM to 10 nM. The two progeny antibodies ADI-09112 and ADI-09113 were used in the second round of affinity optimization.

The second round of affinity optimization: The second step was to improve the affinity of the two anti-PCSK9 monoclonal antibodies ADI-09112 and ADI-09113 (named "parent" antibody) with human-murine cross-activity and ligand competition. In short, a secondary yeast-based antibody presentation library was established again for each parent antibody. The CDR-H3 and light chains (LC) of the parent antibodies were combined with CDR-H1 and CDR-H2 of genes in an existing yeast library (named "H1/H2" optimization). Finally, 5 libraries with a size of about 1 × 10⁸ were generated for the subsequent enriching of antibodies with higher affinity. The screening method was the same as that in the first round of screening. After 2 to 3 rounds of enriching, the yeasts were plated to give monoclonal antibodies. After this, progeny antibodies with improved affinity were obtained, among which ADI-10085, ADI-10086 and ADI-10087 were variants of CDR-H1 and CDR-H2 regions of ADI-09912, and ADI-10088, ADI-10089 and ADI-10090 were variants of VH regions of ADI-09113. The related sequence information of the antibodies is shown in Tables A-C. The affinity of these antibodies for human PCSK9 was increased by 10 times, and the K_{D} ranges were between 4-17 pM and 200 pM (Table 1 and Table 2), as detected by the ForteBio method and MSD-SET assay. The affinity of part of the antibodies was about ten times higher than that of control antibodies. The identification of other antibody functions can further reduce the number of antibodies so as to facilitate preclinical development.

The sequence information and number of each anti-PCSK9 antibody involved herein are shown in Tables A-C below:

**Table A. Sequence number of each CDR of exemplary antibodies disclosed herein**

| | Heavy chain CDR SEQ ID NO | | | Light chain CDR SEQ ID NO | | |
|---|---|---|---|---|---|---|
| IgG ADI name | CDR1 | CDR2 | CDR3 | CDR1 | CDR2 | CDR3 |
| ADI-02396 | 1 | 2 | 3 | 4 | 5 | 6 |
| ADI-09111 | 1 | 2 | 18 | 4 | 5 | 6 |
| ADI-09112 | 1 | 14 | 19 | 4 | 5 | 6 |
| ADI-09113 | 7 | 15 | 18 | 4 | 5 | 6 |
| ADI-10085 | 8 | 16 | 19 | 4 | 5 | 6 |
| ADI-10086 | 9 | 17 | 19 | 4 | 5 | 6 |
| ADI-10087 | 10 | 17 | 19 | 4 | 5 | 6 |
| ADI-10088 | 11 | 17 | 18 | 4 | 5 | 6 |
| ADI-10089 | 12 | 17 | 18 | 4 | 5 | 6 |
| ADI-10090 | 13 | 17 | 18 | 4 | 5 | 6 |

**Table B. Sequence number of each framework region of heavy chain variable regions and light chain variable regions of exemplary antibodies disclosed herein**

| | Framework region of heavy chain variable region SEQ ID NO | | | | Framework region of light chain variable region SEQ ID NO | | | |
|---|---|---|---|---|---|---|---|---|
| IgG ADI name | FR1 | FR2 | FR3 | FR4 | FR1 | FR2 | FR3 | FR4 |
| ADI-02396 | 45 | 46 | 47 | 48 | 49 | 50 | 51 | 52 |
| ADI-09111 | 45 | 46 | 47 | 48 | 49 | 50 | 51 | 52 |
| ADI-09112 | 45 | 46 | 47 | 48 | 49 | 50 | 51 | 52 |
| ADI-09113 | 45 | 46 | 47 | 48 | 49 | 50 | 51 | 52 |
| ADI-10085 | 45 | 46 | 47 | 48 | 49 | 50 | 51 | 52 |
| ADI-10086 | 45 | 46 | 47 | 48 | 49 | 50 | 51 | 52 |
| ADI-10087 | 45 | 46 | 47 | 48 | 49 | 50 | 51 | 52 |
| ADI-10088 | 45 | 46 | 47 | 48 | 49 | 50 | 51 | 52 |
| ADI-10089 | 45 | 46 | 47 | 48 | 49 | 50 | 51 | 52 |
| ADI-10090 | 45 | 46 | 47 | 48 | 49 | 50 | 51 | 52 |

**Table C. Sequence numbers of heavy chain variable regions, light chain variable regions, heavy chain and light chain sequences of exemplary antibodies disclosed herein**

| IgG ADI name | Heavy chain variable region SEQ ID NO | Heavy chain SEQ ID NO | Light chain variable region SEQ ID NO | Light Chain SEQ ID NO |
|---|---|---|---|---|
| ADI-02396 | 23 | 34 | 24 | 35 |
| ADI-09111 | 25 | 36 | 24 | 35 |
| ADI-09112 | 26 | 37 | 24 | 35 |
| ADI-09113 | 27 | 38 | 24 | 35 |
| ADI-10085 | 28 | 39 | 24 | 35 |
| ADI-10086 | 29 | 40 | 24 | 35 |
| ADI-10087 | 30 | 41 | 24 | 35 |
| ADI-10088 | 31 | 42 | 24 | 35 |
| ADI-10089 | 32 | 43 | 24 | 35 |
| ADI-10090 | 33 | 44 | 24 | 35 |

**Table 1: K_{D} of antibodies measured by ForteBio method**

| Sample ID | ForteBio: IgG K_{D} (M), IgG was fixed on a probe | | |
|---|---|---|---|
| | Human PCSK-9 | Cynomolgus monkey PCSK-9 | Murine PCSK-9 |
| ADI-02396 | 9.50E-09 | 3.23E-08 | 7.83 E-08 |
| ADI-09111 | 1.17E-09 | 2.61E-09 | 1.05 E-08 |
| ADI-09112 | 4.97E-10 | 9.83E-10 | 3.78E-09 |
| ADI-09113 | 8.04E-10 | 1.12E-09 | 3.10E-09 |
| ADI-10085 | 2.95E-10 | 3.77E-10 | 5.44E-10 |
| ADI-10086 | 3.36E-10 | 4.57E-10 | 6.07E-10 |
| ADI-10087 | 2.96E-10 | 3.96E-10 | 5.58E-10 |
| ADI-10088 | 4.71E-10 | 6.32E-10 | 8.55E-10 |
| ADI-10089 | 3.11E-10 | 4.08E-10 | 5.89E-10 |
| ADI-10090 | 3.52E-10 | 4.69E-10 | 6.74E-10 |
| Alirocumab | 3.01E-11 | 3.83E-10 | 2.66E-09 |
| Evolocumab | 1.03E-09 | 1.30E-09 | 1.14E-07 |
| Bococizumab | 3.86E-10 | 4.76E-10 | 6.63E-10 |
| Lodelcizumab | 1.51E-09 | 2.34E-09 | NB |

**Table 2: K_{D} of antibodies measured kinetically by MSD-SET**

| Sample ID | MSD: Fab K_{D} (M), IgG was fixed on an assay plate | | | |
|---|---|---|---|---|
| | Human PCSK-9 (pH 7.4) | Human PCSK-9 (pH 6.0) | Cynomolgus monkey PCSK-9 (pH 7.4) | Murine PCSK-9 (pH 7.4) |
| ADI-02396 | ND | ND | 5.90E-09 | ND |
| ADI-09111 | 7.00E-10 | 1.90 E-9 | 1.20E-09 | 3.50 E-09 |
| ADI-09112 | 2.20E-10 | 4.90E-10 | 3.00E-10 | 1.20E-09 |
| ADI-09113 | 1.70E-10 | 6.50 E-10 | 2.40E-10 | 7.60E-10 |
| ADI-10085 | 4.20E-12 | 5.90E-12 | 1.20E-11 | 4.10E-11 |
| ADI-10086 | 8.60E-12 | 1.30 E-11 | 2.40E-11 | 6.00E-11 |
| ADI-10087 | 5.00E-12 | 9.80E-12 | 1.30E-11 | 4.10E-11 |
| ADI-10088 | 1.00E-11 | 1.6E-11 | 2.20E-11 | 8.70E-11 |
| ADI-10089 | 1.10E-11 | 1.00E-11 | 2.20E-11 | 7.00E-11 |
| ADI-10090 | 1.70E-11 | 2.10E-11 | 3.80E-11 | 1.20E-10 |
| Alirocumab | 7.20E-11 | 5.10E-11 | 8.20E-11 | 3.00E-10 |
| Evolocumab | 1.60E-11 | ND | 8.20E-12 | ND |
| Bococizumab | 2.10E-11 | 3.80E-11 | 2.60E-11 | 6.50E-11 |
| Lodelcizumab | ND | ND | 1.00E-10 | NB |

### Example 3. Experiment on Inhibition of the binding of PCSK-9 to LDLR by anti-PCSK9 antibodies

The PCSK9 protein (biotin-labeled PCSK9 protein) described in example 1 was diluted to 400 nmol/L with PBS solution (phosphate-buffered saline solution) to serve as a working solution. The anti-PCSK9 antibodies (ADI-10085, ADI-10086, ADI-10087, ADI-10088, ADI-10089 and ADI-10090) obtained in example 2 were diluted with PBS solution to concentrations of 1000 nmol/L, 100 nmol/L, 10 nmol/L, 1 nmol/L and 0. 1 nmol/L, respectively, and according to the same method, solutions of control antibodies (Alirocumab, Evolocumab, Bococizumab and Lodelcizumab) at each concentration were prepared. The PCSK9 working solution was mixed with an equal volume of each gradiently diluted anti-PCSK9 antibody sample or control sample. CHO cells (CHO-LDLR) overexpressing human LDLR were resuspended in PBS solution and counted. The cell concentration was adjusted to 4 × 10⁶ cells/mL with PBS solution. The CHO cells were inoculated into a U-bottom 96-well plate, with 50 µL of cell medium added in each well. 50 µL of a mixed solution of PCSK9 and each anti-PCSK9 antibody was added. Each well contains 2.0 × 10⁵ cells. The mixture was centrifuged at 200 g at room temperature for 5 min, and the supernatant was discarded. Anti-His-FITC (R&D Systems) was diluted with PBS solution at a ratio of 1:200 to a final concentration of 2.5 µg/mL, added to a 96-well plate, at 100 µL per well, and put in an ice bath for 30 min. Centrifugation was performed at 200 g at room temperature for 5 min, the supernatant was gently discarded, and 150 µL of PBS solution was added into each well; then centrifugation was performed at 200 g at room temperature for 5 min, and the supernatant was gently discarded. This operation was repeated 3 times. PBS solution was added to each well at 80 µL/well, and the cells were pipetted for several times with a pipette for resuspension. The fluorescence signal value of cells was detected by a flow cytometer.

For the fluorescence signals detected in the experiment, see Table 3 below.

**Table 3: Fluorescence signal value**

| Sample name | 1000 nM | 100 nM | 10 nM | 1nM | 0.1 nM |
|---|---|---|---|---|---|
| ADI-10085 | 5700.0 | 5087.0 | 5684.0 | 8914.0 | 10431.0 |
| ADI-10086 | 5269.0 | 5129.0 | 5830.0 | 6919.0 | 6600.0 |
| ADI-10087 | 5094.0 | 5259.0 | 5743.0 | 6483.5 | 6865.0 |
| ADI-10088 | 5216.0 | 5162.0 | 5826.0 | 6827.0 | 6798.0 |
| ADI-10089 | 5331.0 | 5137.0 | 5635.0 | 6500.0 | 6889.5 |
| ADI-10090 | 4822.0 | 4806.0 | 5807.0 | 6750.0 | 6807.0 |
| ADI-02396 | 5993.0 | 5067.0 | 5513.0 | 6375.0 | 6646.0 |
| IgG | 8919.0 | 7523.0 | 6955.0 | 6728.5 | 6998.5 |
| Alirocumab | 4926.0 | 4952.0 | 5748.0 | 6985.0 | 7174.5 |
| Evolocumab | 4805.0 | 5097.0 | 5780.0 | 6807.0 | 6986.0 |
| Bococizumab | 6038.0 | 5307.0 | 6267.5 | 6959.0 | 7096.0 |
| Lodelcizumab | 7330.0 | 8756.0 | 7330.5 | 8756.0 | 8405.0 |
| +PCSK9 | 9977.0 | N/A | N/A | N/A | N/A |
| -PSCK9 | 3985.0 | N/A | N/A | N/A | N/A |
| Blank control | 3782.0 | N/A | N/A | N/A | N/A |

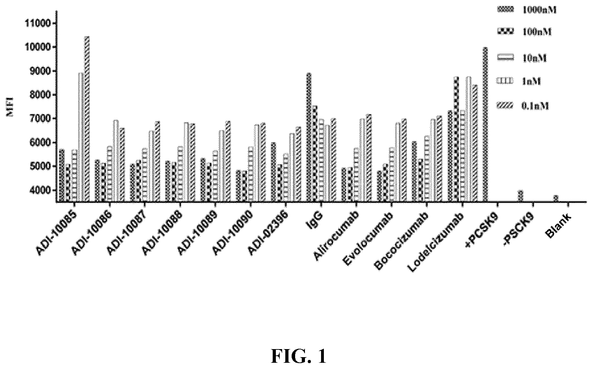

The raw data in Table 3 were analyzed and plotted using GraphPad Prism6, obtaining Figure 1. According to the results, the anti-PCSK9 antibodies have a capability of blocking the binding of PCSK9 to LDLR equivalent to that of the control antibodies.

### Example 4. Analytical Experiment on Uptake of LDL-C by Cells

A HepG2 cell cryopreservation tube was taken out of a liquid nitrogen storage tank and rapidly thawed at 37 °C under a water bath. The cell suspension was transferred into a 15 mL centrifuge tube, and 4 mL of room-temperature growth medium (90% DMEM + 10 % FBS, both purchased from Gibco) was slowly added. After 5 min of centrifugation at 1000 r/min at room temperature, the deposited cells were resuspended in fresh growth medium, transferred into a culture flask, and cultured at 37 °C, 5% CO₂. HepG2 cells in the log phase were washed twice with PBS solution and then digested for 3 min with 1 mL of 0.25% trypsin (purchased from Gibco), and the cells were resuspended in 6 mL of growth medium to quench the reaction. After the cell concentration was adjusted to 0.8 × 10⁶ cells /mL with the growth medium, the HepG2 cells were inoculated into a black clear-bottom 96-well plate coated with poly-D-lysine (purchased from Nunc) at 100 µL/well, and incubated in an incubator at 37 °C, 5% CO₂ for 6 to 7 h. The growth medium was discarded and replaced by assay medium (DMEM + 5% FBS) at 100 µL/well, and the HepG2 cells were cultured in an incubator at 37 °C, 5% CO₂ overnight. The antibody samples (ADI-10085, ADI-10087, ADI-10088 and ADI-10089) were diluted to 66.7 nmol/L with the assay medium. With 66.7 nmol/L as the initial concentration, 4-fold gradient dilution was performed in the same manner for both the positive control antibodies (Alirocumab, Evolocumab and Lodelcizumab) and the negative control antibodies (LDL, PCSK9 + LDL and IgG). 60 µL of each obtained concentration-gradient sample was mixed with an equal volume (60 µL) of 66.7 nmol/L PCSK9, thus obtaining each mixture. The blank control was 120 µL of assay medium. The liquid in the 96-well plate was pipetted out, and 50 µL of the mixture and blank control was added into each well and incubated in an incubator at 37 °C, 5% CO₂ for 1 h. The culture plate was taken out, added with 6 µg/mL BODIPY-labeled LDL solution (purchased from life technologies) diluted with the assay medium at 50 µL/well, and cultured at 37 °C, 5% CO₂ for 4 h. The medium was discarded, and 100 µL of PBS solution was added to each well to wash the plate and then discarded. After washing twice, each well was added with 100 µL of PBS solution. The fluorescence values were read using a Spectra Max I3 microplate reader.

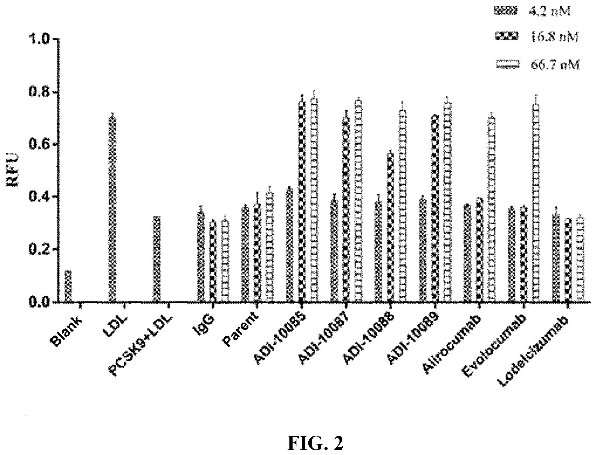

The obtained raw fluorescence data are listed in Table 4 below, which were analyzed and plotted using GraphPad Prism6, thus obtaining FIG. 2. It can be seen from the results that the fluorescence values obtained in the case where HepG2 cells were treated with anti-PCSK9 antibodies (ADI-10085, ADI-10087, ADI-10088, and ADI-10089) were enhanced by about two times compared with the fluorescence values obtained in the absence of the anti-PCSK9 antibodies. These data prove that each anti-PCSK9 antibody disclosed herein can enhance the capability of restoring LDLR in HepG2 cells and inhibit the decrease of LDL-C uptake induced by PCSK9, resulting in increased LDL-C uptake in HepG2 cells, which is superior to the positive control antibodies at gradients of 16.8 nM and 66.7 nM.

**Table 4: Fluorescence signal value**

| Sample name | 4.2 nM | | 16.8 nM | | 66.7 nM | |
|---|---|---|---|---|---|---|
| Blank control | 0.1179 | 0.1174 | N/A | N/A | N/A | N/A |
| LDL | 0.7139 | 0.6903 | N/A | N/A | N/A | N/A |
| PCSK9+LDL | 0.3251 | 0.3259 | N/A | N/A | N/A | N/A |
| IgG | 0.3583 | 0.3250 | 0.2973 | 0.3093 | 0.3288 | 0.2881 |
| ADI-02396 | 0.3662 | 0.3522 | 0.4035 | 0.3410 | 0.4319 | 0.4006 |
| ADI-10085 | 0.4228 | 0.4349 | 0.7800 | 0.7423 | 0.7492 | 0.7973 |
| ADI-10087 | 0.3701 | 0.4030 | 0.7198 | 0.6835 | 0.7576 | 0.7755 |
| ADI-10088 | 0.3570 | 0.4005 | 0.5736 | 0.5611 | 0.7059 | 0.7522 |
| ADI-10089 | 0.3803 | 0.3986 | 0.7116 | 0.7064 | 0.7742 | 0.7413 |
| Alirocumab | 0.3636 | 0.3704 | 0.3958 | 0.3919 | 0.6865 | 0.7158 |
| Evolocumab | 0.3506 | 0.3598 | 0.3555 | 0.3630 | 0.7239 | 0.7785 |
| Lodelcizumab | 0.3181 | 0.3523 | 0.3173 | 0.3141 | 0.3296 | 0.3124 |

### Example 5. Analytical Experiment on Internalization of LDLR into Cells

PCSK9 can directly bind to LDLR to promote the internalization of LDLR which is transported to lysosomes for degradation after entering hepatocytes, thus reducing LDLR expressed on the surface of cells and increasing the level of LDL-C in serum. Anti-PCSK9 antibodies can block the binding of PCSK9 to LDLR, thus reducing the ability of PCSK9 to consume LDLR. In this experiment, the bioactivity of anti-PCSK9 antibodies against the internalization of LDLR into cells was determined by co-incubating CHO-LDLR cells with the anti-PCSK9 antibodies and a PCSK9 protein solution, detecting the fluorescence values of LDLR with the aforementioned flow cytometer, and comparing the fluorescence values of the anti-PCSK9 antibodies and the positive control antibody (Evolocumab).

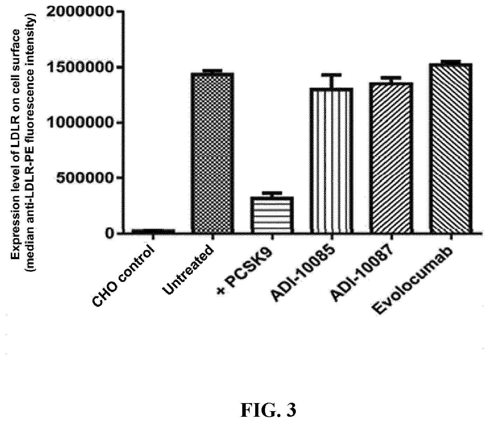

RPMI 1640 cell medium (Gibco) was used to prepare the PCSK9 protein described in example 1 into a concentration of 50 µg/mL. 60 µL of 1000 nm anti-PCSK9 antibodies (ADI-10085 and ADI-10087) were uniformly mixed with a solution of PCSK9 (50 µg/mL) and then the mixture was incubated for 30 min. The positive control antibody was treated in the same way. CHO cells and CHO-LDLR cells were separately centrifuged at 500 g at room temperature for 3 min. The cells were resuspended in PBS solution with the cell density adjusted to 2 × 10⁶ cells/mL. The cell suspensions were added to U-bottom 96-well plates at 100 µL/well. The aforementioned mixed sample was added to the plate at 100 µL/well in quadruplicate, pipetted uniformly, and incubated at 4 °C for 4 h. The mixture was washed 3 times with 200 µL of PBS solution, and centrifuged at 500 g at room temperature for 3 min. 5 µL of anti-LDLR-PE (Sino Biological, Item No. 20131-R301-P) was diluted with 100 µL of PBS solution, and then added to a U-bottom 96-well plate at 100 µL/well and incubated away from light for 30 min. The mixture was washed 3 times with 200 µL of PBS solution, and centrifuged at 500 g for 3 min. The obtained cells were resuspended in cell medium, and the fluorescence signal of the PE fluorescence-labeled LDLR protein on the surface of the CHO-LDLR cells was detected using a flow cytometer. The results are shown in Table 5. The raw data in Table 5 were analyzed and plotted using GraphPad Prism6, obtaining Figure 3.

It can be seen from the results in Table 5 that the antibodies obtained herein effectively block the internalization of LDLR in cells.

**Table 5: Fluorescence signal value**

| Sample name | 1 | 2 | 3 | 4 | **Average** |
|---|---|---|---|---|---|
| CHO control | 26308.5 | 14666.5 | N/A | N/A | 20487.5 |
| Untreated | 1461967 | 1413043 | N/A | N/A | 1437505 |
| + PCSK9 | 267181 | 284270.5 | 353893 | 362791 | 317033.9 |
| ADI-10085 | 1148583 | 1232906 | 1465201 | 1339541 | 1296558 |
| ADI-10087 | 1432592 | 1332187 | 1339160 | 1300079 | 1351005 |
| Evolocumab | 1560702 | 1524849 | 1507073 | 1500379 | 1523251 |

### Example 6. Hypolipidemic Effect of Anti-PCSK9 Antibody on Healthy SD Rats

The tested antibody (anti-PCSK9 antibody ADI-10087) was administered to SPF SD rats (Vital River) according to a conventional method in the art, wherein the female rats were about 254 g to 294 g in weight and about 9 to 12 weeks old, and the male rats were about 369 g to 420 g in weight and about 9 to 12 weeks old. The tested antibody was administered to each group once. The dosage regimen is shown in Table 6.

**Table 6: Dosage design and administration**

| Group No. | Group | Dose (mg/kg) | Concentration (mg/mL) | Administration capacity (mL/kg) | Number of animals |
|---|---|---|---|---|---|
| 1 | Anti-PCSK9 antibody low dose group, single SC administration | 3 | 0.75 | 4 | 6, half female and half male |
| 2 | Anti-PCSK9 antibody medium dose group, single SC administration | 10 | 2.5 | 4 | 6, half female and half male |
| 3 | Anti-PCSK9 antibody high dose group, single SC administration | 30 | 7.5 | 4 | 6, half female and half male |
| 4 | Anti-PCSK9 antibody group, single IV administration | 10 | 2.5 | 4 | 6, half female and half male |
| 5 | Evolocumab control group, single SC administration | 10 | 2.5 | 4 | 6, half female and half male |

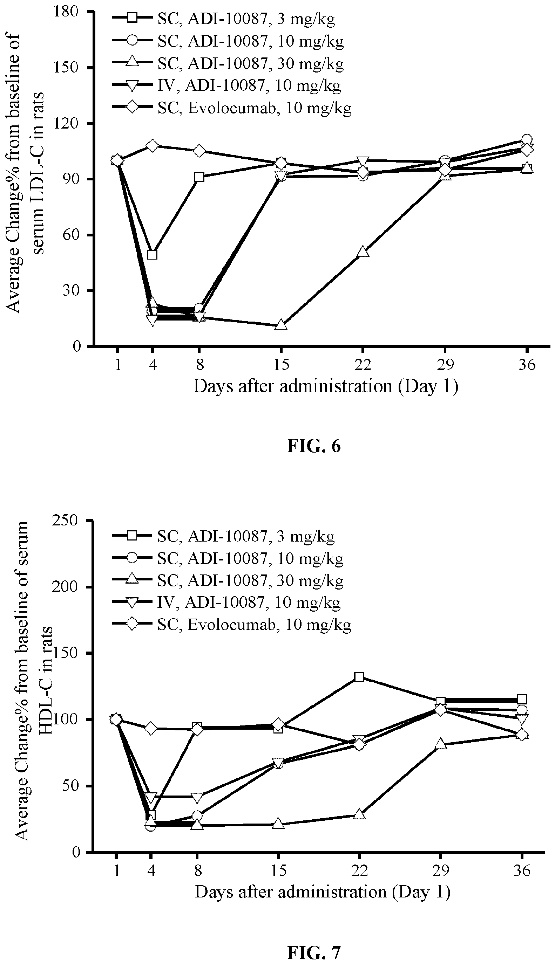

The blood of each group of animals was collected via the jugular vein according to a conventional method at the following time points: 0 h before administration (D1) and 72 h (D4), 168 h (D8), 336 h (D15), 504 h (D22), 672 h (D29), and 840 h (D36) after administration. The blood samples were collected into anticoagulant-free tubes, coagulated on ice and then centrifuged at 2-8 °C at 5000 rpm/min for 10 min, and serum was collected and assayed for LDL-C and HDL-C using a Hitachi-7060 automatic biochemical analyzer. According to the data of blood lipid analysis, the change rate of LDL-C and HDL-C (% LDL-C and % HDL-C) relative to those before administration (baselines) at each time point were calculated. According to the results, it was found that after 3-30 mg/kg of the anti-PCSK9 antibody disclosed herein was subcutaneously administered to the rats once, the LDL-C and HDL-C levels in serum decreased dose-dependently (FIG. 6 and FIG. 7). For example, 3 days, 7 days, 14 days and 21 days after administration, the LDL-C and HDL-C levels significantly decreased in comparison with the baseline levels. In addition, it was also found that after 10 mg/kg of Evolocumab was subcutaneously administered to the rats once, the LDL-C and HDL-C levels in serum did not significantly decrease.

For other antibodies disclosed herein, the same method can also be applied for the above assay.

### Example 7. Hypolipidemic Effect of Anti-PCSK9 Antibody on Healthy Cynomolgus Monkeys

The tested antibody (anti-PCSK9 antibody ADI-10087) was administered to cynomolgus monkeys (Guangdong Blooming-Spring Biological Technology Development Co., Ltd.) according to a conventional method in the art, wherein the female animals were about 2 kg to 4 kg in weight and about 3 to 5 years old, and the male animals were about 3 kg to 5 kg in weight and about 3 to 5 years old. The dosage regimen is shown in Table 7, wherein the tested antibody was administered to groups 1-5 once and group 6 once a week, 4 times in total.

**Table 7: Dosage design and administration**

| Group No. | Group | Dose (mg/kg) | Concentration (mg/mL) | Administration capacity (mL/kg) | Number of animals |
|---|---|---|---|---|---|
| 1 | Anti-PCSK9 antibody low dose group, single SC administration | 3 | 6 | 0.5 | 6, half female and half male |
| 2 | Anti-PCSK9 antibody medium dose group, single SC administration | 10 | 20 | 0.5 | 6, half female and half male |
| 3 | Anti-PCSK9 antibody high dose group, single SC administration | 30 | 60 | 0.5 | 6, half female and half male |
| 4 | Anti-PCSK9 antibody group, single IV administration | 10 | 1 | 10 | 6, half female and half male |
| 5 | Evolocumab control group, single SC administration | 10 | 20 | 0.5 | 6, half female and half male |
| 6 | Anti-PCSK9 antibody group, repeated SC administration | 10 | 20 | 0.5 | 6, half female and half male |

For groups 1-5, blood was collected from the subcutaneous vein or the inguinal femoral artery/inguinal vein in the contralateral forelimb or hind limb of the limb on which the drug was administered according to a conventional method at the following time points: 0 h before administration and 24 h (D2), 72 h (D4), 120 h (D6), 168 h (D8), 336 h (D15), 504 h (D22), 672 h (D29), 840 h (D36), 1008 h (D43), 1176 h (D50) and 1344 h (D57) after administration. With regard to group 6, blood was collected at the following time points according to the aforementioned method: 0 h before the first administration and 24 h (D2), 72 h (D4), 120 h (D6), 168 h (D8, before the second administration) and 336 h (D15, before the third administration) after administration. For the last administration, blood was collected 0 h before administration and 24 h (D2), 72 h (D4), 120 h (D6), 168 h (D8), 336 h (D15), 504 h (D22), 672 h (D29), 840 h (D36), 1008 h (D43), 1176 h (D50) and 1344 h (D57) after administration.

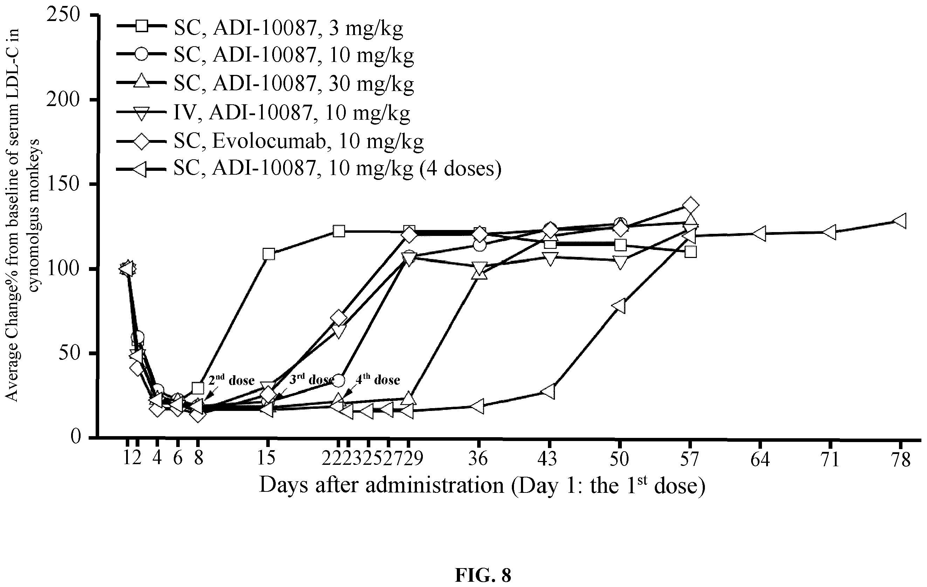

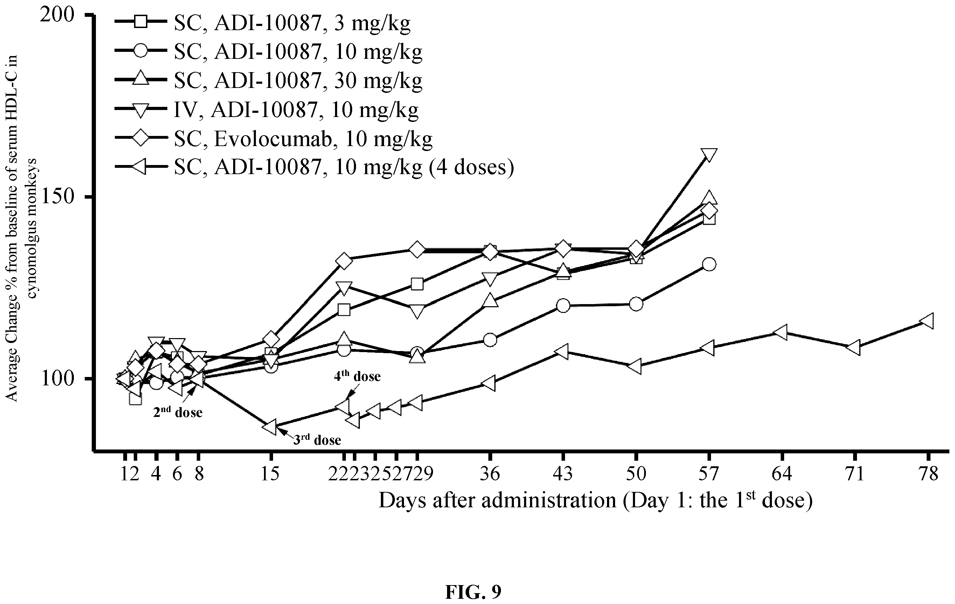

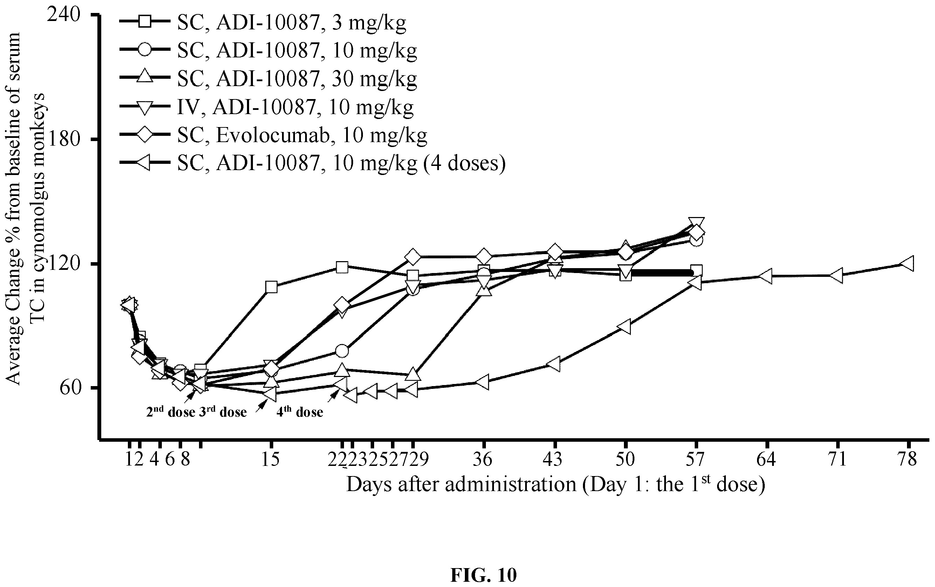

Whole blood was collected into tubes containing coagulant and separation gel, placed on ice for coagulation, and then centrifuged at 2-8 °C at 5000 rpm/min for 10 min, and serum was collected. After all blood samples were collected, total cholesterol (TC), LDL-C and HDL-C were assayed. According to the data of blood lipid analysis, the change rate of LDL-C and HDL-C (% LDL-C and % HDL-C) relative to those before administration (baselines) at each time point were calculated. According to the results, it was found that after a single subcutaneous administration of the anti-PCSK9 antibody (ADI-10087) disclosed herein to the cynomolgus monkeys at 3 mg/kg, 10 mg/kg and 30 mg/kg, both the LDL-C (FIG. 8) and TC (FIG. 10) levels in serum significantly decreased in an obvious dose-effect manner, and were significantly lower than the baseline levels 3 to 28 days after the administration. This indicates that the antibody disclosed herein can effectively alleviate conditions and/or disorders related to LDL-C and TC, such as lowering blood lipid level. The administration of the anti-PCSK9 antibody had no significant influence on the HDL-C level in the serum of the cynomolgus monkeys on the whole (FIG. 9).

However, it was surprisingly found that after separate single subcutaneous administration of the anti-PCSK9 antibody disclosed herein and Evolocumab to the cynomolgus monkeys at 10 mg/kg, Evolocumab only significantly lowered LDL-C in comparison with the baseline level in 14 days after the administration, shorter than 21 days after the administration achieved by the same dose of the antibody disclosed herein. That is, the anti-PCSK9 antibody disclosed herein significantly lowered LDL-C for a longer period than Evolocumab.

For other antibodies disclosed herein, the same method can also be applied for the above assay.

### Example 8. Preparation of the Liquid Preparation Comprising Anti-PCSK9 Antibody (Preparation A) Disclosed Herein

A liquid preparation comprising an anti-PCSK9 antibody with a concentration of 150 mg/mL prepared according to formula A

### Formula A

| | |
|---|---|
| Anti-PCSK9 antibody (ADI-10087) | 150 mg/mL |
| Histidine | 1.5 g/L |
| Arginine | 90 mmol/L |
| Sorbitol | 3% |
| Polysorbate 80 | 0.3 g/L |
| pH | 5.5 |

The anti-PCSK9 antibody (ADI-10087) obtained in example 2 was added to an ultrafiltration centrifuge tube (molecular weight cutoff: 30 KD), after concentration by centrifugation, the anti-PCSK9 antibody was diluted with an aqueous solution of formula A without polysorbate 80 and the anti-PCSK9 antibody (1.5 g/L histidine, 90 mmol/L arginine, 3% sorbitol, pH 5.5) and then concentrated; and this operation was repeated until the replacement was completed. The concentration of the replaced protein was adjusted to 150 mg/mL. Then an aqueous solution of polysorbate 80 (30 g/L, 1/100 by volume) was added to adjust the final concentration of polysorbate 80 to 0.3 g/L. After being aseptically filtered, the semi-finished product was aliquoted into vials and the vials were then capped with rubber stoppers and aluminum-plastic caps, and the product was obtained.

### Example 9. Preparation of Comparative Example (Preparation B) of the Liquid Preparation Comprising Anti-PCSK9 Antibody Disclosed Herein

Comparative example: An preparation comprising anti-PCSK9 antibody liquid with a concentration of 150 mg/mL prepared according to formula B

### Formula B

| | |
|---|---|
| Anti-PCSK9 antibody (ADI-10087) | 150 mg/mL |
| Histidine | 1.5 g/L |
| Arginine | 180 mmol/L |
| Polysorbate 80 | 0.3 g/L |
| pH | 5.5 |

The anti-PCSK9 antibody (ADI-10087) obtained in example 2 was added to an ultrafiltration centrifuge tube (molecular weight cutoff: 30 KD), after concentration by centrifugation, the anti-PCSK9 antibody was diluted with an aqueous solution of formula B without polysorbate 80 and the anti-PCSK9 antibody (1.5 g/L histidine, 180 mmol/L arginine, pH 5.5) and then concentrated; and this operation was repeated until the replacement was completed. The concentration of the replaced protein was adjusted to 150 mg/mL. Then an aqueous solution of polysorbate 80 (30 g/L, 1/100 by volume) was added to adjust the final concentration of polysorbate 80 to 0.3 g/L. After being aseptically filtered, the semi-finished product was aliquoted into vials and the vials were then capped with rubber stoppers and aluminum-plastic caps, and the product was obtained.

### Example 10. Measurement of Viscosity of the Liquid Preparation Comprising Anti-PCSK9 Antibody

The viscosity was measured with a cone-and-plate viscometer (Brookfield, CAP1000+/2000+. For details, visit http://www.sinoinstrument.com/product_details-4-83-401-60.html) at room temperature of 20-25 °C. A rotor and a rotational speed were selected (rotor: cp-40; rotational speed: 25 rpm). The rotor was connected to a connecting nut, with the position adjusted, and samples were added. The Run button was pushed to start the measurement of the viscosity of the samples. According to the above experiment, the viscosity of preparation A was 5.2 centipoises, and the viscosity of preparation B was 6.0 centipoises. Surprisingly, the viscosity of preparation A was significantly lower than that of preparation B, indicating that the combination of arginine and sorbitol is more beneficial to reduce the viscosity of a high-concentration antibody preparation.

### Example 11. Change in Protein Purity of the Liquid Preparation Comprising Anti-PCSK9 Antibody

In order to inspect the stability of preparation A and preparation B, an accelerated stability test was carried out. The product of preparation A and preparation B aliquoted in the plugged and capped vials were examined for accelerated stability at 40 °C ± 2 °C/60% RH ± 5% RH for 2 months. Meanwhile, the change in protein purity was assayed by SEC-HPLC and non-reduced CE-SDS as follows.

### SEC-HPLC:

The sample was diluted to a concentration of 2 mg/mL; a hydrophilic silica gel size exclusion chromatography column TSKG 3000 SWxl was used; the amount of injected protein was 100 µg; the mobile phases were 20 mmol/L Na₂HPO₄•12 H₂O, 150 mmol/L NaCl, and 200 mmol/L arginine; the pH was 6.8; the flow velocity was 0.5 mL/min; the detection wavelength was 280 nm; the column temperature was 25 °C; and the area normalization method was used for calculation.

### CE-SDS:

About 100 µg of sample was taken and added with a sample buffer (pH 7.0) to adjust the total volume to 95 µL. The reduced sample was added with 5 µL of β-mercaptoethanol, and the non-reduced sample was added with 5 µL of 250 mmol/L NEM. After mixing, the mixture was heated at 70 °C for 10 min and detected.

By SEC-HPLC, based on the percentage decrease of an SEC main peak, the changes in protein purity were assayed to be a decrease by 2.6% after 1 month and a decrease by 3.7% after 2 months for preparation A; and a decrease by 4.1% after 1 month and a decrease by 7.0% after 2 months for preparation B. Based on the percentage decrease of non-reduced CE-SDS, the changes in protein purity were assayed to be a decrease by 0.4% after 2 weeks and a decrease by 2.0% after 1 month for preparation A; and a decrease by 2.5% after 2 weeks and a decrease by 7.3% after 1 month for preparation B. It can be seen that the purity change rate of preparation A is significantly slower than that of preparation B. Relative to preparation B, preparation A has higher stability.

### Example 12. Assay of Charge Heterogeneity of the Liquid Preparation Comprising Anti-PCSK9 Antibody

At different time points, preparation A and preparation B which had undergone the accelerated stability test in example 11 were sampled for charge heterogeneity assay. Beckman uncoated capillaries and a Beckman PA800 plus capillary electrophoresis apparatus were adopted; the sampling voltage was 0.5 psi; the sampling time was 10.0 s; the separation voltage was 30 kV; and the analysis time was 30 min. The area normalization method was used to calculate the peak area of acidic components, basic components, and the main peak as a percentage of the total peak area. The CZE main peak of preparation A dropped by 0% after 2 weeks and by 2.9% after 1 month. The CZE main peak of preparation B dropped by 2.0% after 2 weeks and by 7.6% after 1 month.

It can be seen that the charge heterogeneity change rate of preparation A is significantly slower than that of preparation B, thus the combination of arginine and sorbitol contributes to less charge heterogeneity change of the preparation.

## Claims

1. A liquid antibody preparation, comprising:
(i) an anti-PCSK9 antibody or an antigen-binding fragment thereof;
(ii) a buffer;
(iii) a viscosity inhibitor selected from sugar alcohols, arginine, arginine hydrochloride, sodium thiocyanate, ammonium thiocyanate, ammonium sulfate, ammonium chloride, calcium chloride, zinc chloride, sodium acetate and combinations thereof; and
(iv) a surfactant,
wherein the anti-PCSK9 antibody or the antigen-binding fragment thereof comprises a heavy chain and a light chain, wherein
(a) the heavy chain comprises or consists of an amino acid sequence set forth in SEQ ID NO: 41;
and
(b) the light chain comprises or consists of an amino acid sequence set forth in SEQ ID NO: 35.

2. The liquid antibody preparation according to claim 1, wherein the concentration of the anti-PCSK9 antibody or the antigen-binding fragment thereof in the liquid antibody preparation is about 50 mg/mL to about 200 mg/mL, preferably about 100 mg/mL to about 200 mg/mL.

3. The liquid antibody preparation according to claim 1 or claim 2, wherein the concentration of the buffer in the liquid antibody preparation is about 0.01-50 mg/mL, for example, about 0.1-50 mg/mL.

4. The liquid antibody preparation according to any of claims 1 to 3, wherein the buffer is selected from histidine, glutamate, phosphate, acetate, citrate and tris(hydroxymethyl)aminomethane buffers, preferably histidine buffer.

5. The liquid antibody preparation according to any of claims 1 to 4, wherein the concentration of the viscosity inhibitor is about 10 mmol/L to 1000 mmol/L, for example, about 20 mmol/L to 500 mmol/L.

6. The liquid antibody preparation according to any of claims 1 to 5, wherein the viscosity inhibitor is sorbitol.

7. The liquid antibody preparation according to claim 6, wherein the viscosity inhibitor is sorbitol with a concentration of about 1% to 6% (w/v).

8. The liquid antibody preparation according to any of claims 1 to 5, wherein the viscosity inhibitor is a combination of sorbitol and arginine or an arginine salt, and the arginine salt is preferably arginine hydrochloride.

9. The liquid antibody preparation according to claim 8, wherein the concentration of the sorbitol is about 1% to 6% (w/v), preferably about 2% to 4% (w/v), and more preferably about 3% (w/v), and the concentration of the arginine or an arginine salt is about 50 mmol/L to 180 mmol/L, preferably about 70 mmol/L to 150 mmol/L, and more preferably about 90 mmol/L.

10. The liquid antibody preparation according to any of claims 1 to 9, wherein the concentration of the surfactant is about 0.01 mg/mL to 5 mg/mL, for example, about 0.05 mg/mL to 1 mg/mL.

11. The liquid antibody preparation according to any of claims 1 to 10, wherein the surfactant is a nonionic surfactant, such as pluronics, polysorbate-80, polysorbate-60, polysorbate-40, or polysorbate-20.

12. The liquid antibody preparation according to any of claims 1 to 11, wherein the pH of the liquid preparation is about 5.0 to 6.0, for example, about 5.2 to 5.8.

13. The liquid antibody preparation according to any of claims 1 to 12, wherein the viscosity of the liquid preparation at 25 °C is about 1.0 to 20 centipoises, for example, about 1.0 to 10 centipoises.

14. The liquid antibody preparation according to claim 13, wherein the liquid preparation is a pharmaceutical preparation, preferably an injection, and more preferably a subcutaneous injection.

15. A solid preparation, obtained by lyophilizing the liquid antibody preparation according to any of claims 1 to 14.

16. The liquid antibody preparation according to any of claims 1 to 14 for use in treating cholesterol-related diseases.

17. Use of sugar alcohol as a viscosity inhibitor in a liquid preparation comprising an anti-PCSK9 antibody, wherein the liquid preparation comprising an anti-PCSK9 antibody is the liquid preparation according to any of claims 1 to 14 and wherein, preferably, the sugar alcohol is sorbitol.

18. Use of sugar alcohol as a viscosity inhibitor in preparing a liquid preparation comprising an anti-PCSK9 antibody, wherein the liquid preparation comprising an anti-PCSK9 antibody is the liquid preparation according to any of claims 1 to 14, and wherein, preferably, the sugar alcohol is sorbitol.

19. Use of a combination of sugar alcohol and arginine or arginine salt, preferably arginine hydrochloride, as a viscosity inhibitor in a liquid preparation comprising an anti-PCSK9 antibody, wherein the liquid preparation comprising an anti-PCSK9 antibody is the liquid preparation according to any of claims 1 to 14, and wherein, preferably, the sugar alcohol is sorbitol.

20. Use of a combination of sugar alcohol and arginine or arginine salt, preferably arginine hydrochloride, as a viscosity inhibitor in preparing a liquid preparation comprising an anti-PCSK9 antibody, wherein the liquid preparation comprising an anti-PCSK9 antibody is the liquid preparation according to any of claims 1 to 14, and wherein, preferably, the sugar alcohol is sorbitol.

## Patentansprüche

1. Flüssige Antikörperzubereitung, umfassend:
(i) einen Anti-PCSK9-Antikörper oder ein antigenbindendes Fragment davon;
(ii) einen Puffer;
(iii) einen Viskositätsinhibitor ausgewählt aus Zuckeralkoholen, Arginin, Argininhydrochlorid, Natriumthiocyanat, Ammoniumthiocyanat, Ammoniumsulfat, Ammoniumchlorid, Calciumchlorid, Zinkchlorid, Natriumacetat und Kombinationen davon; und
(iv) ein Tensid,
wobei der Anti-PCSK9-Antikörper oder das Antigen-bindende Fragment davon eine schwere Kette und eine leichte Kette umfasst, wobei
(a) die schwere Kette eine in SEQ ID NO: 41 dargelegte Aminosäuresequenz umfasst oder daraus besteht;
und
(b) die leichte Kette eine in SEQ ID NO: 35 dargelegte Aminosäuresequenz umfasst oder daraus besteht.

2. Flüssige Antikörperzubereitung nach Anspruch 1, wobei die Konzentration des Anti-PCSK9-Antikörpers oder des antigenbindenden Fragments davon in der flüssigen Antikörperzubereitung etwa 50 mg/ml bis etwa 200 mg/ml, vorzugsweise etwa 100 mg/ml bis etwa 200 mg/ml, beträgt.

3. Flüssige Antikörperzubereitung nach Anspruch 1 oder Anspruch 2, wobei die Konzentration des Puffers in der flüssigen Antikörperzubereitung etwa 0,01 bis 50 mg/ml, beispielsweise etwa 0,1 bis 50 mg/ml, beträgt.

4. Flüssige Antikörperzubereitung nach einem der Ansprüche 1 bis 3, wobei der Puffer ausgewählt ist aus Histidin-, Glutamat-, Phosphat-, Azetat-, Citrat- und Tris(hydroxymethyl)aminomethan-Puffern, vorzugsweise Histidin-Puffer.

5. Flüssige Antikörperzubereitung nach einem der Ansprüche 1 bis 4, wobei die Konzentration des Viskositätsinhibitors etwa 10 mmol/L bis 1000 mmol/L, beispielsweise etwa 20 mmol/L bis 500 mmol/L, beträgt.

6. Flüssige Antikörperzubereitung nach einem der Ansprüche 1 bis 5, wobei der Viskositätsinhibitor Sorbit ist.

7. Flüssige Antikoerperzubereitung nach Anspruch 6, wobei der Viskositätsinhibitor Sorbit mit einer Konzentration von etwa 1 % bis 6 % (w/v) ist.

8. Flüssige Antikörperzubereitung nach einem der Ansprüche 1 bis 5, wobei der Viskositätsinhibitor eine Kombination aus Sorbit und Arginin oder ein Argininsalz ist und das Argininsalz vorzugsweise Argininhydrochlorid ist.

9. Flüssige Antikörperzubereitung nach Anspruch 8, wobei die Konzentration des Sorbits etwa 1 % bis 6 % (w/v), vorzugsweise etwa 2 % bis 4 % (w/v) und besonders bevorzugt etwa 3 % (w/v) beträgt und die Konzentration des Arginins oder eines Argininsalzes etwa 50 mmol/L bis 180 mmol/L, vorzugsweise etwa 70 mmol/L bis 150 mmol/L und besonders bevorzugt etwa 90 mmol/L beträgt.

10. Flüssige Antikörperzubereitung nach einem der Ansprüche 1 bis 9, wobei die Konzentration des Tensids etwa 0,01 mg/ml bis 5 mg/ml, beispielsweise etwa 0,05 mg/ml bis 1 mg/ml, beträgt.

11. Flüssige Antikörperzubereitung nach einem der Ansprüche 1 bis 10, wobei das Tensid ein nichtionisches Tensid ist, wie Pluronics, Polysorbat-80, Polysorbat-60, Polysorbat-40 oder Polysorbat-20.

12. Flüssige Antikörperzubereitung nach einem der Ansprüche 1 bis 11, wobei der pH-Wert der flüssigen Zubereitung etwa 5,0 bis 6,0, beispielsweise etwa 5,2 bis 5,8, beträgt.

13. Flüssige Antikörperzubereitung nach einem der Ansprüche 1 bis 12, wobei die Viskosität der flüssigen Zubereitung bei 25 °C etwa 1,0 bis 20 Centipoise, beispielsweise etwa 1,0 bis 10 Centipoise, beträgt.

14. Flüssige Antikörperzubereitung nach Anspruch 13, wobei die flüssige Zubereitung eine pharmazeutische Zubereitung, vorzugsweise eine Injektion, und besonders bevorzugt eine subkutane Injektion ist.

15. Feste Zubereitung, erhalten durch Lyophilisieren der flüssigen Antikörperzubereitung nach einem der Ansprüche 1 bis 14.

16. Flüssige Antikörperzubereitung nach einem der Ansprüche 1 bis 14 zur Verwendung bei der Behandlung von cholesterinbedingten Erkrankungen.

17. Verwendung von Zuckeralkohol als Viskositätsinhibitor in einer flüssigen Zubereitung, umfassend einen Anti-PCSK9-Antikörper, wobei die flüssige Zubereitung, umfassend einen Anti-PCSK9-Antikörper, die flüssige Zubereitung nach einem der Ansprüche 1 bis 14 ist und wobei, vorzugsweise, der Zuckeralkohol Sorbit ist.

18. Verwendung von Zuckeralkohol als Viskositätsinhibitor beim Zubereiten einer flüssigen Zubereitung, umfassend einen Anti-PCSK9-Antikörper, wobei die flüssige Zubereitung, umfassend einen Anti-PCSK9-Antikörper, die flüssige Zubereitung nach einem der Ansprüche 1 bis 14 ist und wobei, vorzugsweise, der Zuckeralkohol Sorbit ist.

19. Verwendung einer Kombination aus Zuckeralkohol und Arginin oder Argininsalz, vorzugsweise Argininhydrochlorid, als Viskositätsinhibitor in einer flüssigen Zubereitung, umfassend einen Anti-PCSK9-Antikörper, wobei die flüssige Zubereitung, umfassend einen Anti-PCSK9-Antikörper, die flüssige Zubereitung nach einem der Ansprüche 1 bis 14 ist, und wobei der Zuckeralkohol vorzugsweise Sorbit ist.

20. Verwendung einer Kombination aus Zuckeralkohol und Arginin oder Argininsalz, vorzugsweise Argininhydrochlorid, als Viskositätsinhibitor beim Zubereiten einer flüssigen Zubereitung, umfassend einen Anti-PCSK9-Antikörper, wobei die flüssige Zubereitung, umfassend einen Anti-PCSK9-Antikörper, die flüssige Zubereitung nach einem der Ansprüche 1 bis 14 ist, und wobei der Zuckeralkohol vorzugsweise Sorbit ist.

## Revendications

1. Préparation d'anticorps liquide, comprenant :
(i) un anticorps anti-PCSK9 ou un fragment de liaison à l'antigène de celui-ci ;
(ii) un tampon ;
(iii) un inhibiteur de viscosité choisi parmi des alcools de sucre, l'arginine, le chlorhydrate d'arginine, le thiocyanate de sodium, le thiocyanate d'ammonium, le sulfate d'ammonium, le chlorure d'ammonium, le chlorure de calcium, le chlorure de zinc, l'acétate de sodium et leurs combinaisons ; et
(iv) un tensioactif ;
dans laquelle l'anticorps anti-PCSK9 ou le fragment de liaison à l'antigène de celui-ci comprend une chaîne lourde et une chaîne légère, dans laquelle
(a) la chaîne lourde comprend ou est constituée d'une séquence d'acides aminés définie dans SEQ ID NO : 41 ; et
(b) la chaîne légère comprend ou est constituée d'une séquence d'acides aminés définie dans SEQ ID NO : 35.

2. Préparation d'anticorps liquide selon la revendication 1, dans laquelle la concentration de l'anticorps anti-PCSK9 ou du fragment de liaison à l'antigène de celui-ci dans la préparation d'anticorps liquide est d'environ 50 mg/ml à environ 200 mg/ml, de préférence d'environ 100 mg/ml à environ 200 mg/ml.

3. Préparation d'anticorps liquide selon la revendication 1 ou la revendication 2, dans laquelle la concentration du tampon dans la préparation d'anticorps liquide est d'environ 0,01 à 50 mg/ml, par exemple d'environ 0,1 à 50 mg/ml.

4. Préparation d'anticorps liquide selon l'une quelconque des revendications 1 à 3, dans laquelle le tampon est choisi parmi des tampons histidine, glutamate, phosphate, acétate, citrate et tris(hydroxyméthyl)aminométhane, de préférence un tampon histidine.

5. Préparation d'anticorps liquide selon l'une quelconque des revendications 1 à 4, dans laquelle la concentration de l'inhibiteur de viscosité est d'environ 10 mmol/l à 1000 mmol/l, par exemple d'environ 20 mmol/l à 500 mmol/l.

6. Préparation d'anticorps liquide selon l'une quelconque des revendications 1 à 5, dans laquelle l'inhibiteur de viscosité est le sorbitol.

7. Préparation d'anticorps liquide selon la revendication 6, dans laquelle l'inhibiteur de viscosité est le sorbitol avec une concentration d'environ 1 % à 6 % (p/v).

8. Préparation d'anticorps liquide selon l'une quelconque des revendications 1 à 5, dans laquelle l'inhibiteur de viscosité est une combinaison de sorbitol et d'arginine ou d'un sel d'arginine, et le sel d'arginine est de préférence le chlorhydrate d'arginine.

9. Préparation d'anticorps liquide selon la revendication 8, dans laquelle la concentration du sorbitol est d'environ 1 % à 6 % (p/v), de préférence d'environ 2 % à 4 % (p/v), et de manière davantage préférée d'environ 3 % (p/v), et la concentration de l'arginine ou d'un sel d'arginine est d'environ 50 mmol/l à 180 mmol/l, de préférence d'environ 70 mmol/l à 150 mmol/l, et de manière davantage préférée d'environ 90 mmol/l.

10. Préparation d'anticorps liquide selon l'une quelconque des revendications 1 à 9, dans laquelle la concentration du tensioactif est d'environ 0,01 mg/ml à 5 mg/ml, par exemple d'environ 0,05 mg/ml à 1 mg/ml.

11. Préparation d'anticorps liquide selon l'une quelconque des revendications 1 à 10, dans laquelle le tensioactif est un tensioactif non ionique, tel que le pluronics, le polysorbate-80, le polysorbate-60, le polysorbate-40 ou le polysorbate-20.

12. Préparation d'anticorps liquide selon l'une quelconque des revendications 1 à 11, dans laquelle le pH de la préparation liquide est d'environ 5,0 à 6,0, par exemple d'environ 5,2 à 5, 8 .

13. Préparation d'anticorps liquide selon l'une quelconque des revendications 1 à 12, dans laquelle la viscosité de la préparation liquide à 25 °C est d'environ 1,0 à 20 centipoises, par exemple d'environ 1,0 à 10 centipoises.

14. Préparation d'anticorps liquide selon la revendication 13, dans laquelle la préparation liquide est une préparation pharmaceutique, de préférence une injection, et de manière davantage préférée une injection sous-cutanée.

15. Préparation solide, obtenue par lyophilisation de la préparation d'anticorps liquide selon l'une quelconque des revendications 1 à 14.

16. Préparation d'anticorps liquide selon l'une quelconque des revendications 1 à 14 pour une utilisation dans le traitement de maladies liées au cholestérol.

17. Utilisation d'alcool de sucre comme inhibiteur de viscosité dans une préparation liquide comprenant un anticorps anti-PCSK9, dans laquelle la préparation liquide comprenant un anticorps anti-PCSK9 est la préparation liquide selon l'une quelconque des revendications 1 à 14 et dans laquelle, de préférence, l'alcool de sucre est le sorbitol.

18. Utilisation d'alcool de sucre comme inhibiteur de viscosité dans la préparation d'une préparation liquide comprenant un anticorps anti-PCSK9, dans laquelle la préparation liquide comprenant un anticorps anti-PCSK9 est la préparation liquide selon l'une quelconque des revendications 1 à 14, et dans laquelle, de préférence, l'alcool de sucre est le sorbitol.

19. Utilisation d'une combinaison d'alcool de sucre et d'arginine ou de sel d'arginine, de préférence le chlorhydrate d'arginine, comme inhibiteur de viscosité dans une préparation liquide comprenant un anticorps anti-PCSK9, dans laquelle la préparation liquide comprenant un anticorps anti-PCSK9 est la préparation liquide selon l'une quelconque des revendications 1 à 14, et dans laquelle, de préférence, l'alcool de sucre est le sorbitol.

20. Utilisation d'une combinaison d'alcool de sucre et d'arginine ou de sel d'arginine, de préférence le chlorhydrate d'arginine, comme inhibiteur de viscosité dans la préparation d'une préparation liquide comprenant un anticorps anti-PCSK9, dans laquelle la préparation liquide comprenant un anticorps anti-PCSK9 est la préparation liquide selon l'une quelconque des revendications 1 à 14, et dans laquelle, de préférence, l'alcool de sucre est le sorbitol.
